# EUROPEAN PATENT APPLICATION

(11) **EP 4 060 045 A1**
(43) Date of publication of application: **21.09.2022**
(21) Application number: 21305319.2
(22) Date of filing: 16.03.2021
(51) Int. Cl.: C12N 15/82

(54) **A GENETICALLY ENGINEERED PLANT OR PART THEREOF ADAPTED FOR THE PRODUCTION OF RECOMBINANT PROTEINS AND PROCESS THEREOF**

(71) Applicant: Core Biogenesis, 91058 Evry (FR)
(72) Inventor: MEZIADI, Chouaib, 91058 Evry (FR)
(74) Representative: Atout PI Laplace

(57) **Abstract**

The invention relates to a genetically engineered plant or part thereof adapted for the production of recombinant proteins, the plant comprising at least one inactivated gene involved in the Transcriptional Gene Silencing (TGS) and/or Post Transcriptional Gene Silencing (PTGS) mechanism and at least one nucleic acid construct comprising a viral promoter coupled to a nucleic acid construct comprising a coding sequence of a recombinant protein. The invention also relates a nucleic acid construct, a process for producing a recombinant protein, a method for obtaining a genetically engineered plant or part thereof and a fusion protein comprising a recombinant protein and an oil-body protein produced by, or obtained from the genetically engineered plant or new-grown plants from the seeds of the genetically engineered plant.

## Description

The sequence listing associated with this application is provided in text format in lieu of a paper copy, and is hereby incorporated by reference into the specification. The name of the text file containing the Sequence Listing is "072797 EP Sequences_ST25.txt".

### FIELD OF THE INVENTION

The present invention relates to a genetically engineered plant or part thereof adapted for the production of recombinant proteins, the plant comprising at least one inactivated gene involved in the Transcriptional Gene Silencing (TGS) and/or Post Transcriptional Gene Silencing (PTGS) mechanism and at least one nucleic acid construct comprising a strong promoter coupled to a nucleic acid construct comprising a coding sequence of a recombinant protein.

The present invention also relates to a nucleic acid construct, a process for producing a recombinant protein, a method for obtaining a genetically engineered plant or part thereof and a fusion protein comprising a recombinant protein and an oil-body protein produced by, or obtained from the genetically engineered plant or new-grown plants from the seeds of the genetically engineered plant.

### BACKGROUND PRIOR ART

A recombinant protein is a protein produced by cells whose DNA has been modified by genetic engineering. Recombinant proteins can be molecules of pharmaceutical, therapeutic, cosmetic interest, molecules used in research and development or in the field of agroindustry. So as to be produced in large quantities, the host cell whose DNA has been modified must grow in large quantities and the protein produced must be functional and easy to purify. In particular, the recombinant protein coding sequence must be implemented in the host cell in a large number of copies with a promoter which allows an important rate of transcription or which can be induced.

Up to now, available molecules are mainly produced in bacteria, mammalian cells and, to a lesser extent, in yeasts and insect cells. These conventional cell culture-based systems are useful but limited by high cost, low efficiency or low yield. Besides, mammalian cells as production hosts raise safety and ethical concerns. Therefore, global production capacities for recombinant proteins are currently insufficient or see their field of applications limited.

Plants as recombinant protein production hosts have been considered as a viable option. Indeed, they provide a valuable alternative to fermentation-based systems for the production of recombinant proteins, having numerous advantages. First, they have the ability to perform post-translational modifications such as correct folding, glycosylation and disulfide bonds that are essential for most mammalian proteins' activity. Particularly, such post-translational modifications are essential for proteins to be effective. Without such post-translational modifications, recombinant proteins obtained may not be functional and artificially reproducing these post-translational modifications may be considered impossible at the moment or too costly. Secondly, there is a greatly reduced risk of contamination with human pathogens, which is a serious concern with mammalian cells in bioreactors. Thirdly, plant growth requirements are simple and not expensive compared to traditional fermentation-based systems, allowing for low-cost scale-up. Whole plants have quite simple growth needs compared to mammalian cell cultures as they only need light as an energy source and minerals to grow. In addition, transient expression systems are able to produce proteins including vaccine candidates in high quantity within a short time period (4-6 weeks), an efficient time scale in case of pandemics or flu epidemics (Gleba Y, Klimyuk V, Marillonnet S. Magnifection--a new platform for expressing recombinant vaccines in plants. Vaccine. 2005 Mar 18;23(17-18):2042-8. doi: 10.1016/j.vaccine.2005.01.006. PMID: 15755568; Musiychuk, Konstantin et al. "A launch vector for the production of vaccine antigens in plants." Influenza and other respiratory viruses vol. 1, 1 (2007): 19-25. doi:10.1111/j.1750-2659.2006.00005.x).

To obtain genetically engineered plants, solutions such as stable nuclear transformation may be used, which is a flexible solution with efficient scale-up. Plastid genome engineering may also be used as their highly polyploid and circular DNA allows a large number of transgene copies. However, plastidial expression is prokaryotic in nature and cannot do post-translational modifications described earlier. Transient expression using agroinfiltration also allows production of a recombinant protein with a maximum yield between 2 and 5 days post infiltration.

The main drawback of the stable nuclear transformation system is low expression levels which allows between 0,001% and 1% of TSP (Total Soluble Proteins) mainly due to gene silencing by Transcriptional Gene Silencing (TGS) and posttranscriptional gene silencing (PTGS). These production rates are far from being enough to compete with traditional fermentation-based systems. Low levels of protein accumulation also increase the cost of purification to non-economic levels. For agroinfiltration, in addition to the need of setting up a complex and costly system to produce on a large scale, transgene RNAs are frequently recognized by plant cells as being "foreign" and silenced by post-transcriptional gene silencing (PTGS). Even if this PTGS can be overcome in some cases by co-expressing a viral suppressor of gene silencing, such as the p19 protein of tomato bushy stunt virus (TBSV), the production in agroinfiltrated leaves remains between 1% TSP and 15% TSP. Further, this method is time-consuming, its implementation requires expert manipulations, and the extraction and purification process is as expensive as conventional systems.

One of the major criteria for high-level expression of a protein is the use of a strong promoter. Viral promoters are by far the most used ones. However, the stronger the expression, the more irregularity is observed. This is mainly due to a defence mechanism called gene silencing triggered against the expression of these transgenes which are treated like invasive nucleic acids due to their high level expression under the strong promoter. Gene silencing can be activated against the introduced gene or against its transcripts by the transcriptional or post-transcriptional gene silencing (PTGS and TGS) respectively. This inactivation corresponds to the specific degradation of messenger RNA (mRNA) encoded by the transgene and the production of double-stranded RNAs (dsRNA) by RNA-DEPENDANT RNA POLYMERASES (RDRs). Then, dsRNAs are processed into 21 to 24 nt (nucleotides) duplexes according to the DICER-LIKE (DCL) enzyme and are protected from exonucleases through 2'O-methylation by HUA ENHANCER 1 (HEN1). Finally, one strand of the small RNA duplexes is loaded into ARGONAUTE (AGO) proteins therefore forming the RNA-inducing silencing complex (RISC) which will drive sequence-specific mRNA cleavage & translational repression or RNA directed DNA methylation (RdDM)(Martínez de Alba AE, Elvira-Matelot E, Vaucheret H. Gene silencing in plants: a diversity of pathways. Biochim Biophys Acta. 2013 Dec; 1829(12):1300-8. doi: 10.1016/j.bbagrm.2013.10.005. Epub 2013 Nov 1. PMID: 24185199).

Several strategies can be adopted to avoid this phenomenon, for example by expressing simultaneously a silencing suppressor in addition to the gene of interest. These suppressors generally have a viral origin. For example p19 protein from the Tomato Bushy Stunt Virus (TBSV) is a suppressor which can be co-expressed with a recombinant protein which results in a highly increased expression level of the protein of interest (Voinnet et al. Suppression of gene silencing: a general strategy used by diverse DNA and RNA viruses of plants. Proc Natl Acad Sci USA. 1999). However, such suppressors of gene silencing can affect the development of the plant and exhibit heterogeneity when they are co-infiltrated in transient expression.

Ultimately, the cost of the purification process with plants could be identical to the one used in fermentation-based systems and onerous steps of extraction-purification of the recombinant protein from plant tissue are required. Often, the recombinant protein is inserted into the matrix of plant tissue, which makes its extraction and purification difficult to carry out, even though this step must be carried out as quickly as possible due to the risk of proteolysis in plant extracts. In conventional systems, recombinant proteins are extracted along with many other compounds such as nucleic acids, chlorophyll, alkaloids, phenolics, polysaccharides and proteases. The problem is that these compounds can not only bind and degrade the recombinant protein but also foul chromatography resins and crossflow membranes resulting in reducing final product quality and yield or adding to manufacturing costs.

Therefore, global production capacities for recombinant proteins are currently insufficient or see their field of applications limited. Hence there is a need to find alternative solutions and/or innovative technologies.

The invention seeks to overcome the aforementioned drawbacks of the prior art as it aims to provide a genetically engineered plant which allows producing large quantities of recombinant proteins which are functional, can be easily extracted and purified, and which may be used for multiple applications.

### SUMMARY OF THE INVENTION

To this effect, the present invention relates to a genetically engineered plant or part thereof adapted for the production of recombinant proteins comprising:
- at least one inactivated gene involved in the Transcriptional Gene Silencing (TGS) and/or Post Transcriptional Gene Silencing (PTGS) mechanism corresponding to :
   (1) at least one knocked-out gene involved in the TGS and/or PTGS mechanism; and/or
   (2) at least one nucleic acid RNA interference construct targeting the RNA molecules expressed from the at least one gene involved in the TGS and/or PTGS mechanism;
- at least one nucleic acid construct comprising a strong promoter coupled to a polynucleotide sequence comprising a coding sequence of a recombinant protein;
wherein (i) the coding sequence of the recombinant protein is linked in a reading frame to an oil body protein coding sequence; and/or (ii) the at least one inactivated gene involved in the TGS and/or PTGS mechanism includes at least one inactivated gene involved in the TGS mechanism and at least one inactivated gene involved in the PTGS mechanism.

By "at least one inactivated gene" as used herein it is understood a gene which cannot encode an RNA or a protein or encodes no functional protein, or which encodes a protein with reduced activity by at least 70 %, preferably at least 90 %. Said "inactivated gene" can encode a protein with reduced functionality, or be a fully inactivated gene, encoding no functional protein or not encoding any protein. An inactivated gene involved in the TGS or PTGS mechanism in the present invention is usually a wild-type gene, i.e. a wild-type gene which, due to the genetically engineered inactivation, cannot encode an RNA or protein, or encodes no functional protein, or encodes a protein with reduced activity, by at least 70 %, preferably at least 90 %.

By "gene involved in the Transcriptional Gene Silencing (TGS) and/or Post Transcriptional Gene Silencing (PTGS) mechanism", it is particularly understood as any gene involved in the TGS and/or PTGS mechanism which would prevent the transcription or the RNA product of the genetically engineered at least one nucleic acid construct, which comprises a strong promoter coupled to a polynucleotide sequence comprising a coding sequence of a recombinant protein, wherein such coding sequence of a recombinant protein may be linked in a reading frame to an oil body protein coding sequence.

By "knocked-out gene", it is understood a gene which has been genetically engineered and which has at least one genetic alteration in its coding sequence which leads to the inability to encode an RNA or a protein or which leads to a protein which is not functional, or with a reduced activity, by at least 70%. By genetic alteration, it is intended to signify at least one among the following modifications: an insertion of a stop codon, an insertion of a nucleotide resulting in the creation of a stop codon, a shift of the nucleotide reading frame resulting in a protein which is not functional, the introduction of a recombination site, such as Cre-Lox, or any modification which has a highly suppressive effect of at least 70%, preferably at least 90% on the expression of the target gene. Other modifications altering the coding sequence of the gene so as to knock it out may also be used. Such genetic alterations typically result from the use of a method selected among CRISPR-Cas 9, synthetic RNAi, TALENs, Meganuclease, TILLING and Zinc finger nuclease but other methods may be suitable. As an alternative, other methods such as physical and chemical mutagenesis by Gamma irradiation or EMS treatment may be used. Preferably, the method used for generating the genetic alterations is CRISPR-Cas9.

By "at least one nucleic acid RNA interference construct targeting the RNA molecules expressed from the at least one gene involved in the TGS and/or PTGS mechanism", it is understood that the at least one gene involved in the TGS and/or PTGS mechanism allows generation of RNAi duplex, generated from the natural TGS and PTGS mechanism or by a hairpin artificial structure.

By "at least one nucleic acid construct comprising a strong promoter coupled to a polynucleotide sequence comprising a coding sequence of a recombinant protein", it is intended to signify a genetically engineered construct which has been inserted into the plant so as to enable the plant to produce the recombinant protein. The said 'at least one nucleic acid construct' may also be referred to as a transgene. By "at least one nucleic acid construct comprising a strong promoter coupled to a polynucleotide sequence comprising a coding sequence of a recombinant protein linked in a reading frame to an oil body protein coding sequence", it is intended to signify a genetically engineered construct which has been inserted into the plant so as to enable the plant to produce the recombinant protein and the oil body protein.

By "oil body protein", it is understood as a protein which may be part of a lipid-containing structure which is typically intracellular. Typically, a lipid-containing structure occurring in plants comprising triacylglycerol molecules or other neutral lipids surrounded by a phospholipid half-unit membrane and oil body proteins.

Regarding oil-body proteins, the water insoluble oil fraction is stored in discrete subcellular structures variously known in the art as oil bodies which may be referred as oleosomes, lipid bodies, lipid droplets or spherosomes. Besides a mixture of oils, such as triacylglycerides or other neutral lipids, which chemically are defined as glycerol or fatty alcohol esters of fatty acids, oil bodies comprise phospholipids and a number of associated proteins, collectively termed oil body proteins.

In the present invention "a strong promoter" is used. It will be understood that the strength of a promoter is determined in part by the attributes of the cell in which it operates. By "a strong promoter", it is intended to mean a promoter which results in high-level expression of gene elements, which causes mRNAs to be initiated at high frequencies. The term "strong promoter" is a term well known and widely used in the art and many strong promoters are known in the art, or can be identified by routine experimentation. It may be a native promoter or a promoter which is not native of the engineered plant, such as another plant promoter, a viral promoter, a bacterial promoter or even a synthetic promoter. Each of these promoters may be used in any embodiment disclosed herein. Preferably, the strong promoter is a viral promoter. By "a viral promoter", "a bacterial promoter", "a synthetic promoter", it is intended to mean any viral, bacterial, synthetic promoter which may be used in plants.

For instance, as viral promoters, the promoter from CMV (P CMV) the mtHP promoter (Xiao, K., Zhang, C., Harrison, M., & Wang, Z. (2004). Isolation and characterization of a novel plant promoter that directs strong constitutive expression of transgenes in plants. Molecular Breeding, 15, 221-231; Patent EP1608761B1), the MMV FLt promoter (Dey, N., Maiti, I.B. Structure and promoter/leader deletion analysis of mirabilis mosaic virus (MMV) full-length transcript promoter in transgenic plants. Plant Mol. Biol. 40, 771-782 (1999); Patent US6420547B1), the rbcS1 promoter (Outchkourov NS, Peters J, de Jong J, Rademakers W, Jongsma MA. The promoter-terminator of chrysanthemum rbcS1 directs very high expression levels in plants. Planta. 2003 Apr) and the Chlorella virus gene promoter (Mitra A, Higgins DW, Rohe NJ. A Chlorella virus gene promoter functions as a strong promoter both in plants and bacteria. Biochem Biophys Res Commun. 1994 Oct 14) may be used. Also, the 35 S viral promoter may be used, for instance CaMV 35S or its derivatives including the tandem sequence. Also double 35S CaMV or CSMV promoter may be used.

Advantageously, the at least one inactivated gene involved in the TGS or PTGS mechanism is chosen among the Dicer-like (DCL) family, the Suppressor of Gene Silencing (SGS) family, the Argonaute (AGO) family, the RNA dependent RNA polymerase (RdR) family and combinations thereof.

Advantageously, the at least one inactivated gene involved in the TGS or PTGS mechanism includes at least one inactivated gene involved in the TGS mechanism and at least one inactivated gene involved in the PTGS mechanism. For instance, the at least one gene involved in the TGS mechanism may be RDR2 or DCL3 or one of AGOs genes. For instance the at least one gene involved in the PTGS mechanism may be RDR6 or DCL2 or DCL4.

Advantageously, the at least one inactivated gene involved in the TGS and/or PTGS mechanism includes the RdR2 gene and the RdR6 gene.

Advantageously, the oil body protein coding sequence is the coding sequence of an oil body protein selected among oleosin, caleosin, steroleosin, perilipin or caveolin.

The coding sequence of the recombinant protein in the at least one nucleic acid construct is not limited. The coding sequence of the recombinant protein may be related to proteins used in cosmetic industry, pharmaceutical industry, food industry or agriculture. Advantageously, the coding sequence of the recombinant protein may be selected among the coding sequence of antibodies, growth factors, enzymes, structural proteins, receptors and signaling proteins and antigens.

Advantageously, the coding sequence of a recombinant protein is the coding sequence or a partial coding sequence of a protein or epitope of a virus. Preferably, such virus is a virus causing human disease. Even more preferably, such protein or epitope of a virus is a protein or epitope of the SARS-CoV-2 virus, for instance the peplomers or Spike protein of SARS-CoV-2.

Advantageously, the plant is a genetically engineered oleaginous plant or part thereof, preferably selected among the following genus: *Arachis, Camelina Theobroma, Brassica, Gossypium, Olea, Ricinus, Glycine, Helianthus, Elaeis, Carthamus* and *Linum.*

Alternatively, the plant is a genetically engineered *Nicotiana spp including Nicotiana benthamiana* or *Camelina sativa* or part thereof. A genetically engineered plant being a double mutant RdR2 RdR6 of the *Nicotiana benthamiana* and *Camelina sativa* species is particularly adapted for the expression of the nucleic acid construct comprising a strong promoter coupled to a polynucleotide sequence comprising a coding sequence of a recombinant protein in reading frame to an oil body protein coding sequence.

Advantageously, the part thereof is at least one part selected among: the seeds, the pollens, the flowers, the roots, the stems, the spores, the mega- or micro-spores, embryos and the vegetative organs of non-flowering plants.

The present invention also relates to a nucleic acid construct comprising a strong promoter coupled to a polynucleotide sequence comprising a coding sequence of a recombinant protein linked in a reading frame to an oil body protein coding sequence.

Advantageously, the oil body protein coding sequence is a coding sequence of oleosin, caleosin, steroleosin, perilipin or caveolin.

Advantageously, the strong promoter of the nucleic acid construct may be a viral promoter such as described previously.

The coding sequence of the recombinant protein in the at least one nucleic acid construct is not limited. The coding sequence of the recombinant protein may be related to proteins used in cosmetic industry, pharmaceutical industry, food industry or agriculture. Advantageously, the coding sequence of the recombinant protein is selected among the coding sequence of antibodies, growth factors, enzymes, structural proteins, receptors and signaling proteins and antigens.

The present invention also relates to a process for producing a recombinant protein in a genetically engineered plant or part thereof, comprising the steps of :
- inactivating at least one gene involved in the TGS and/or PTGS mechanism in a plant or part thereof by:
   (1) generating at least one genetic alteration in the coding sequence of the at least one gene involved in the TGS and/or PTGS mechanism,
      such generating step being performed by using a method selected among: CRISPR-Cas 9, TALENs, Meganuclease, TILLING, Zinc finger nuclease, synthetic RNAi, physical or chemical mutagenesis and combinations thereof; and/or
   (2) inserting in the plant at least one nucleic acid RNA interference construct targeting the RNA molecules expressed from the at least one gene involved in the TGS and/or PTGS mechanism;
- inserting in the plant at least one nucleic acid construct comprising a strong promoter coupled to a polynucleotide sequence comprising a coding sequence of a recombinant protein,
   wherein (i) the coding sequence of the recombinant protein is linked in a reading frame to an oil body protein coding sequence; and/or (ii) the step of inactivating at least one gene includes the inactivation of at least one gene involved in the TGS mechanism and at least one gene involved in the PTGS mechanism,
   wherein the step of inserting is performed by using a method selected among: CRISPR-Cas 9, TALENs, Meganuclease, Zinc finger nuclease with particle gun transformation and Agrobacterium tumefaciens,
   wherein the step of inactivating may be performed after the step of inserting, and
- optionally recovering the recombinant protein and the oil body protein and optionally purifying the recombinant protein recovered.

Preferably, the method used for generating the genetic alterations is CRISPR-Cas9.

Advantageously in that process, the at least one inactivated gene involved in the TGS or PTGS mechanism is chosen among the Dicer-like (DCL) family, the Suppressor of Gene Silencing (SGS) family, the Argonaute (AGO) family the RNA dependent RNA polymerase (RdR) family and combinations thereof.

Advantageously, the at least one inactivated gene involved in the TGS or PTGS mechanism includes at least one inactivated gene involved in the TGS mechanism and at least one inactivated gene involved in the PTGS mechanism. For instance, the at least one gene involved in the TGS mechanism may be RDR2 or DCL3 or one of AGOs genes. For instance the at least one gene involved in the PTGS mechanism may be RDR6 or DCL2 or DCL4.

Advantageously in that process, the at least one inactivated gene involved in the TGS and/or PTGS mechanism includes the RdR2 gene and the RdR6 gene.

Advantageously in that process, the oil body protein coding sequence is a coding sequence of oleosin, caleosin, steroleosin, perilipin or caveolin.

The coding sequence of the recombinant protein in the at least one nucleic acid construct is not limited. The coding sequence of the recombinant protein may be related to proteins used in cosmetic industry, pharmaceutical industry, food industry or agriculture. Advantageously in that process, the coding sequence of the recombinant protein is selected among the coding sequence of antibodies, growth factors, enzymes, structural proteins, receptors and signaling proteins and antigens.

Advantageously in that process, the coding sequence of a recombinant protein is the coding sequence or a partial coding sequence of a protein or epitope of a virus. Preferably, such virus is a virus causing human disease. Even more preferably, such protein or epitope of a virus is a protein or epitope of the SARS-CoV-2 virus, for instance the peplomers or Spike protein of SARS-CoV-2

Advantageously in that process, the plant is a genetically engineered oleaginous plant or part thereof, preferably selected among the following genus: *Arachis, Camelina, Theobroma, Brassica, Gossypium, Olea, Ricinus, Glycine, Helianthus, Elaeis, Carthamus* and *Linum.*

Alternatively in that process, the plant is a genetically engineered *Nicotiana spp including Nicotiana benthamiana* or *Camelina sativa* or part thereof.

Advantageously in that process, the part thereof is at least one part selected among: the seeds, the pollens, the flowers, the roots, the stems, the spores, mega- or micro-spores, the embryos and the vegetative organs of non-flowering plants.

The present invention also relates to a method for obtaining a genetically engineered plant or part thereof as described above, the method comprising:
- inactivating at least one gene involved in the TGS and/or PTGS mechanism gene in a plant or part thereof by :
   (1) generating at least one genetic alteration in the coding sequence of one or more gene involved in the TGS and/or PTGS mechanism, such generating step being performed by using a method selected among: CRISPR-Cas 9, TALENs, Meganuclease, TILLING, Zinc finger nuclease, synthetic RNAi, physical or chemical mutagenesis and combinations thereof; and/or
   (2) implementing RNA interference genes targeting the RNA molecules expressed from the TGS and/or PTGS mechanism genes,
      and
- inserting in the plant a nucleic acid construct comprising a strong promoter coupled to a nucleic acid sequence comprising a coding sequence of a recombinant,
   wherein (i) the coding sequence of the recombinant protein is linked in a reading frame to an oil body protein coding sequence; and/or (ii) the step of inactivating at least one gene includes the inactivation of at least one gene involved in the TGS mechanism and at least one gene involved in the PTGS mechanism,
   such step of inserting being performed by using a method selected among: CRISPR-Cas 9, TALENs, Meganuclease, Zinc finger nuclease with particle gun transformation and Agrobacterium tumefaciens,
wherein the inactivation step can be performed after the step of inserting.

Advantageously, the method further comprises:
- collecting the seeds of the genetically engineered plant with at least one inactivated gene involved in the TGS and/or PTGS mechanism and the nucleic acid construct inserted;
- growing new plants from the seeds and/ or plant tissues; and
- selecting the heterozygotes or homozygotes from new-grown plants which have at least one inactivated gene involved in the TGS and/or PTGS mechanism and the nucleic acid construct, and all progeny and genetic crosses using these plants.

The present invention also relates to a fusion protein comprising a recombinant protein and an oil-body protein produced by, or obtained from:
- the genetically engineered plant or part thereof as described above, or
- the genetically engineered plant or part thereof obtained from the method as described above, or
- the heterozygotes or homozygotes new-grown plants selected as described above.

The present invention also relates to a seed from the genetically engineered plant or part thereof as described above or recovered or purified from the process as described above.

The present invention also relates to a callus of the genetically engineered plant of the invention.

Although it is not admitted that an essentially biological process may allow obtaining a genetically engineered plant or part thereof of the present invention, or a nucleic acid construct of the present invention, or a fusion protein comprising a recombinant protein and an oil-body protein produced by, or obtained from the genetically engineered plant or part thereof of the present invention or obtained from the heterozygotes or homozygotes new-grown plants selected of the present invention, it is stated that the claimed products are obtained by means which are not essentially biological process and the claimed methods do not correspond to essentially biological process.

Further characteristics, details and advantages of the invention will emerge from the description made with reference to the annexed drawings given by way of example.

### BRIEF DESCRIPTION OF THE DRAWINGS

The invention will be better understood and its various characteristics and advantages will emerge from the following description of a number of exemplary embodiments and its appended figures in which:
- Figure 1 displays a first diagram representing the TGS and PTGS mechanisms;
- Figure 2 displays a second diagram representing the strategy used according to a particular embodiment of the present invention.

### DETAILED DESCRIPTION OF THE INVENTION

In this specification, the invention will be described by way of examples. However, the invention is not restricted to these examples.

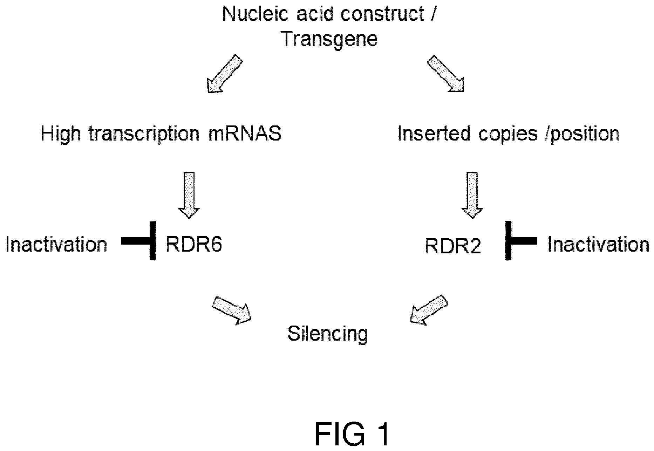

Figure 1 displays a first diagram representing the TGS and PTGS mechanisms. In a classic functioning cell, a double strand DNA (dsDNA) is transcribed by RNA polymerase II in single strand RNAs (ssRNA). Such ssRNAs are then replicated by RdR6 to double strand RNAs (dsRNAs) which mediates the production of 21-22 nucleotides by DCL4 or 2 or 24 nucleotides by DCL3. HEN1 allows the methylation of such nucleotides which are then used as guides by the AGOs proteins for the silencing process. These proteins are components of the RNA-Induced Silencing Complex (RISC) which allows gene silencing, and according to RdR6, the post-transcriptional gene silencing (PTGS) mechanism.

On the other side of figure 1 is shown the similar transcriptional gene silencing (TGS) mechanism which is related to RdR2 and DCL3.

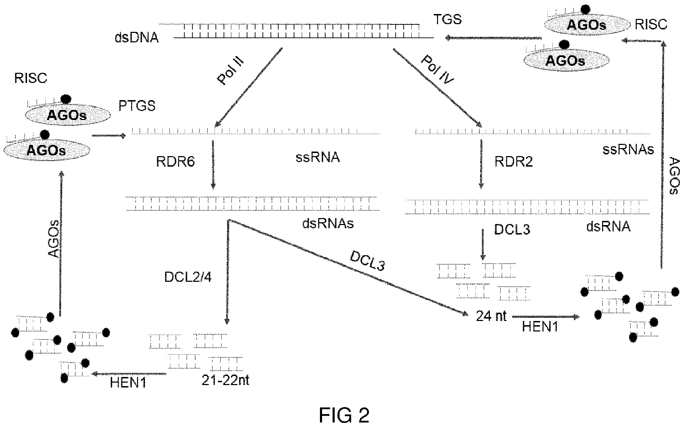

Figure 2 displays a second diagram representing the strategy used according to a particular embodiment of the present invention. In figure 2, the nucleic acid construct, or transgene, which has been inserted in the plant, allows the high transcription of mRNAs as the strong promoter of the nucleic acid construct is coupled to a polynucleotide sequence of a recombinant protein. Moreover, the nucleic acid construct is inserted in multiple copies, at multiple positions. In a plant which has only been engineered by the insertion of the nucleic acid construct, RdR6 would allow the silencing at post transcription level while RdR2 would allow the silencing at transcription level, resulting in the protection of the plant against the expression of the nucleic acid construct.

According to a particular embodiment of the invention, the RdR2 and RdR6 genes are inactivated by the generation of at least one genetic alteration in the coding sequence of the RdR2 and RdR6 genes of the plant cells. Such inactivation prevents the silencing mechanism to be effective against the transcription of the nucleic acid construct inserted. Therefore, the plant cells are used as a production unit so as to obtain the recombinant protein of the recombinant protein coding sequence comprised in the nucleic acid construct.

According to present invention, the nucleic acid construct may comprise coding sequences of the recombinant protein and the oil body protein in a reading frame, allowing the expression of both coding sequences so as to obtain fusion proteins. By "linked in a reading frame" it is understood that the recombinant protein and the oil body protein coding sequences are set on consecutive triplets of DNA, which are non-overlapping. The order coding sequences of the recombinant protein and the oil-body protein is not relevant and they may be inverted.

The fusion protein may correspond to a tandem fusion, with the proteins being connected end-to-end or by protein linkers. Specific protein linkers may be particularly suitable for the purification process and methods of the present invention so as for these protein linkers to be specifically targeted so as to separate the recombinant protein from the oil-body protein. Such protein linkers may correspond to multiple glycine residues or proline residues but other protein linkers may be suitable.

Further, the fusion proteins may accumulate in oil bodies, which may be referred as oleosomes, containing mainly the recombinant protein and the oil body protein, such as oleosin. Other oil body proteins may be used, such as caleosin, steroleosin, perilipin and caveolin. Particularly, the oil bodies are present in the seeds and the leaves.

Among the strong promoters of the nucleic acid construct cited above, viral promoters may be used. For instance, as viral promoters, the promoter from CMV (P CMV) the mtHP promoter (Xiao, K., Zhang, C., Harrison, M., & Wang, Z. (2004). Isolation and characterization of a novel plant promoter that directs strong constitutive expression of transgenes in plants. Molecular Breeding, 15, 221-231; Patent EP1608761B1), the MMV FLt promoter (Dey, N., Maiti, I.B. Structure and promoter/leader deletion analysis of mirabilis mosaic virus (MMV) full-length transcript promoter in transgenic plants. Plant Mol. Biol. 40, 771-782 (1999); Patent US6420547B1), the rbcS1 promoter (Outchkourov NS, Peters J, de Jong J, Rademakers W, Jongsma MA. The promoter-terminator of chrysanthemum rbcS1 directs very high expression levels in plants. Planta. 2003 Apr) and the Chlorella virus gene promoter (Mitra A, Higgins DW, Rohe NJ. A Chlorella virus gene promoter functions as a strong promoter both in plants and bacteria. Biochem. Biophys. Res Commun. 1994 Oct 14) may be used. Also, the 35 S viral promoter may be used, for instance CaMV 35S or its derivatives including the tandem sequence. Also double 35S CaMV or CSMV promoter may be used.

In the genetically engineered plant or part thereof of the present invention, or the associated process and methods, the inactivation of at least one gene involved in the Transcriptional Gene Silencing (TGS) and/or Post Transcriptional Gene Silencing (PTGS) mechanism allows blocking the triggering of silencing against the expression of the at least one nucleic acid which would be considered as invasive nucleic acids due to an high level of expression under a strong promoter such as for instance a viral promoter. Said inactivation prevents the specific degradation of messenger RNA encoded by the nucleic acid construct and/or prevents the production of double stranded RNAs (dsRNA) encoded by the nucleic acid construct. The inactivation may inhibit sequence-specific mRNA cleavage and translational repression or RNA directed DNA methylation.

The inactivation of the said at least one gene involved in the Transcriptional Gene Silencing (TGS) and/or Post Transcriptional Gene Silencing (PTGS) mechanism is not lethal to the genetically engineered plant.

In the present invention the at least one inactivated gene involved in the TGS or PTGS mechanism may be chosen among the Dicer-like (DCL) family, the Suppressor of Gene Silencing (SGS) family, the Argonaute family (AGO), the RNA dependent RNA polymerase (RdR) family and combinations thereof. However, the at least one gene to inactivate may vary depending on the species or genus of plants which is genetically engineered.

In the case of a genetically engineered plant comprising at least one nucleic acid construct comprising a strong promoter coupled to a polynucleotide sequence comprising a coding sequence of a recombinant protein linked in a reading frame to an oil body protein coding sequence, such a genetically engineered plant allows increasing the yield and simplifying the first steps of purification extraction by addressing the recombinant proteins to oil-bodies particularly present in leaves and seeds. Therefore, cell fractionation and purification of lipid bodies carrying the recombinant proteins by flotation gradient may be extracted from the genetically engineered plant or parts thereof and particularly from the leaves or seeds.

According to the alternatives of the present invention, on one hand, with RNAi inhibition modification the genetically engineered plant may be cultivated so as to be more resistant to pathogens. On the other hand, with knocked-out genes, the recombinant protein production yield is greater.

In a particular embodiment, the at least one inactivated gene involved in the TGS or PTGS mechanism includes at least one inactivated gene involved in the TGS mechanism and at least one inactivated gene involved in the PTGS mechanism. For instance, the at least one gene involved in the TGS mechanism may be RDR2 or DCL3 or one of AGOs genes. For instance the at least one gene involved in the PTGS mechanism may be RDR6 or DCL2 or DCL4.

Advantageously, the at least one inactivated gene involved in the TGS and/or PTGS mechanism includes the RdR2 gene and the RdR6 gene. Such genetically engineered plant or part thereof may be referred as a double mutant RdR2 RdR6, which increases the expression of the nucleic acid construct by disrupting homogeneously the silencing mechanism at the transcriptional and post-transcriptional level and without having an impact on plant development. A specific RdR2 RdR6 double mutant which is preferred is a RdR2 RdR6 double mutant of *Nicotiana spp including Nicotiana benthamiana* or *Camelina sativa.*

The coding sequence of the recombinant protein in the at least one nucleic acid construct is not limited. The coding sequence of the recombinant protein may be related to proteins used in cosmetic industry, pharmaceutical industry, food industry or agriculture. Advantageously, the coding sequence of the recombinant protein is selected among the coding sequence of antibodies, growth factors, enzymes, structural proteins, receptors and signaling proteins and antigens.

Advantageously, the coding sequence of a recombinant protein is the coding sequence of a protein or a partial coding sequence of a protein or epitope of a virus. Preferably, such virus is a virus causing human disease. Even more preferably, such protein or epitope of a virus is a protein or epitope of the SARS-CoV-2 virus, for instance the peplomers or Spike protein of SARS-CoV-2

By "optionally recovering the recombinant protein and the oil body protein", it is understood that as soon as a genetically engineered plant or part thereof as indicated above is obtained, the genetically engineered plant or part thereof is able to express the coding sequence of the recombinant protein and the oil body protein by initiating the expression from the strong promoter. Due to a high expression activity caused by the strong promoter, the proteins which will be obtained will be in high quantity and they will gather in oil bodies, particularly in the seeds and in the leaves.

Further when using this approach, the recombinant proteins recovered can be cleaved from the oil bodies as a first purification step, for instance using an endoprotease. Depending on the recombinant protein and the oil-body protein used but also on the type of protein fusion, which may be an end-to-end connection or a protein linker, the cleavage step may be adapted. Further, the process of the invention may comprise the purification of the recombinant protein according to conventional methods.

Examples of plants which may be used are listed: Arabidopsis (*Arabidopsis thaliana*), *Camelina* (*Camelina spp*), Rapeseed (*Brassica* spp.), Soybean (*Glycine max*), Sunflower (*Helianthus annuus*), oil palm (*Elaeis guineeis*), cottonseed (*Gossypium* spp.), groundnut (*Arachis hypogaea),* coconut (*Cocus nucifera*), castor (*Ricinus communis*), Safflower (*Carthamus tinctorius*), mustard (*Brassica* spp. and *Sinapis alba*), coriander (*Coriandrum Sativum*), Squash (*Cucurbita maxima*), linseed/flax (*Linum usitatissimum*), Brazil nut (*Bertholletia excelsa*), jojoba (*Simmondsia chinensis*) and maize (*Zea mays*). However, other plants may be suitable for the present invention.

A list of sequences is provided according to Table 1.

| Table 1 | |
|---|---|
| Sequence number | Title |
| SEQ ID NO 1 | Transcript sequence of RdR2 (AT4G11130.1) / Chr4:6780522..6784440 forward |
| SEQ ID NO 2 | Transcript sequence of RdR2 (LOC104707406) NCBI Reference Sequence: XM_01 0423753.2 |
| SEQ ID NO 3 | Transcript sequence of RdR2-like (LOC104737110) NCBI Reference Sequence: XM_01 0457214.2 |
| SEQ ID NO 4 | Transcript sequence of RdR2-like 2 (LOC104719267) NCBI Reference Sequence: XM_019230033.1 |
| SEQ ID NO 5 | Transcript sequence of RdR6 (AT3G49500.1) / Chr3:18349161..18353624 reverse |
| SEQ ID NO 6 | Transcript sequence of RdR6 (LOC104711463) NCBI Reference Sequence: XM_019229277.1 |
| SEQ ID NO 7 | Transcript sequence of RdR6 variant (LOC104791175) NCBI Reference Sequence: XM_019246751.1 |
| SEQ ID NO 8 | Transcript variant of RdR6-like (LOC104780770) NCBI Reference Sequence: XM_01 0505306.2 |
| SEQ ID NO 9 | Binary vector pLX-CaMV, complete sequence MH200606 REGION: 260..1219 |
| SEQ ID NO 10 | Cloning vector Cloning vector pG2RNAi2, complete sequence / KT954097 REGION: 2921..3429 |
| SEQ ID NO 11 | Example of oleosin fused to a part of the receptor binding domain of the Spike protein of SARS-CoV-2 by the intermediary of the prochymosin sequence |

Seq. 1 is an example of transcript sequence of RdR2 in *Arabidopsis thaliana;* Seq. 2 is an example of transcript sequence of RdR2 in *Camelina sativa;* Seq. 3 is an example of transcript sequence of RdR2-like in *Camelina sativa;* Seq. 4 is another example of transcript sequence of RdR2-like in *Camelina sativa;* Seq. 5 is an example of transcript sequence of RdR6 in *Arabidopsis thaliana;* Seq. 6 is an example of transcript sequence of RdR6 in *Camelina sativa;* Seq. 7 is an example of variant transcript sequence of RdR6 in *Camelina sativa;* Seq. 8 is an example of another variant transcript sequence of RdR6-like in *Camelina sativa;* Seq. 10 is an example of a strong promoter being a Double 35S CaMV promoter; Seq. 11 is an example of a strong promoter being a CSMV promoter; Seq. 11 is an example of an amino acids sequence an oleosin fused to a part of the receptor binding domain of the Spike protein of SARS-CoV-2.

Advantageously, the strong promoter comprises or consists of the sequence of a Double 35S CaMV promoter set forth in SEQ. 9 or a variant thereof with at least 70% identity thereto, preferably 90% identity. Such promoters may be used with any embodiment of the present invention.

Advantageously, the strong promoter comprises or consists of the sequence of a CSMV promoter set forth in SEQ. 10 or a variant thereof with at least 90% identity thereto. Such promoter may be used with any embodiment of the present invention.

Advantageously, the coding sequence of a recombinant protein being the receptor binding domain of the Spike protein of SARS-CoV-2 fused to an oleosin comprises a sequence having at least 90% sequence identity to SEQ. 11 (amino acids) or SEQ. 12 (DNA). Such sequence may be used for the nucleic acid construct comprising a coding sequence of a recombinant protein in a reading frame with an oil-body protein coding sequence of any embodiment of the present invention.A fusion protein which is enzymatically active and resides on the oil bodies may be used directly. The fusion protein may be stable in dry seeds for long periods, such as 2 to 4 years and when extracted has a half-life of 3 to 4 weeks on oil bodies.

The genetically engineered plant or part thereof of the present invention and the methods and process of the present invention allow the production of recombinant proteins with oil bodies and recombinant proteins purified, in a manner which is extremely inexpensive, with plants which may allow switching to open-field production, offering a novel route to the manufacture of recombinant proteins.

The present invention is directed to genetically engineered plants which have been modified locally so as to obtain a transient expression of a recombinant protein. The present invention is also directed to the genetically engineered plants which have been obtained from the seeds of a previous genetically engineered plant according to the invention and which allow a permanent expression of said recombinant protein.

The examples described above are given as illustrations of embodiments of the invention. They do not in any way limit the scope of the invention which is defined by the following claims.

## Claims

1. A genetically engineered plant or part thereof adapted for the production of recombinant proteins comprising:
- at least one inactivated gene involved in the Transcriptional Gene Silencing (TGS) and/or Post Transcriptional Gene Silencing (PTGS) mechanism corresponding to :
(1) at least one knocked-out gene involved in the TGS and/or PTGS mechanism; and/or
(2) at least one nucleic acid RNA interference construct targeting the RNA molecules expressed from the at least one gene involved in the TGS and/or PTGS mechanism;
- at least one nucleic acid construct comprising a strong promoter coupled to a polynucleotide sequence comprising a coding sequence of a recombinant protein;
wherein (i) the coding sequence of the recombinant protein is linked in a reading frame to an oil body protein coding sequence; and/or (ii) the at least one inactivated gene involved in the TGS and/or PTGS mechanism includes at least one inactivated gene involved in the TGS mechanism and at least one inactivated gene involved in the PTGS mechanism.

2. The genetically engineered plant or part thereof according to claim 1, wherein the at least one inactivated gene involved in the TGS or PTGS mechanism is chosen among the Dicer-like (DCL) family, the Suppressor of Gene Silencing (SGS) family, the Argonaute (AGO) family, the RNA dependent RNA polymerase (RdR) family and combinations thereof.

3. The genetically engineered plant or part thereof according to any one of the preceding claims, wherein the at least one inactivated gene involved in the TGS and/or PTGS mechanism includes the RdR2 gene and the RdR6 gene.

4. The genetically engineered plant or part thereof according to any one of the preceding claims, the oil body protein coding sequence being the coding sequence of an oil body protein selected among oleosin, caleosin, steroleosin, perilipin or caveolin.

5. The genetically engineered plant or part thereof according to any one of the preceding claims, wherein the coding sequence of a recombinant protein is selected among the coding sequence of a protein selected among antibodies, growth factors and antigens.

6. The genetically engineered plant or part thereof according to any one of the preceding claims, wherein the coding sequence of a recombinant protein is the coding sequence of a protein of the SARS-CoV-2 virus.

7. The genetically engineered plant or part thereof according to any one of the preceding claims, the plant being a genetically engineered oleaginous plant or part thereof, preferably selected among the following genus: *Arachis, Camelina, Theobroma, Brassica, Gossypium, Olea, Ricinus, Glycine, Helianthus, Elaeis, Carthamus* and *Linum.*

8. The genetically engineered plant or part thereof according to any one of claims 1 to 6, being a genetically engineered *Nicotiana spp and preferably Nicotiana benthamiana* or *Camelina sativa* or part thereof.

9. The genetically engineered plant or part thereof according to any one of the preceding claims, the said part being at least one part selected among: the seeds, the pollens, the flowers, the roots, the stems, the mega- or micro-spores, the embryos and the vegetative organs of non-flowering plants.

10. The genetically engineered plant or part thereof according to any one of the preceding claims, the strong promoter being a viral promoter.

11. A nucleic acid construct comprising a strong promoter coupled to a polynucleotide sequence comprising a coding sequence of a recombinant protein linked in a reading frame to an oil body protein coding sequence.

12. A process for producing a recombinant protein in a genetically engineered plant or part thereof, comprising the steps of :
- inactivating at least one gene involved in the TGS and/or PTGS mechanism in a plant or part thereof by :
(1) generating at least one genetic alteration in the coding sequence of the at least one gene involved in the TGS and/or PTGS mechanism, such generating step being performed by using a method selected among: CRISPR-Cas 9, TALENs, Meganuclease, TILLING, Zinc finger nuclease, synthetic RNAi, physical or chemical mutagenesis and combinations thereof; and/or
(2) inserting in the plant at least one nucleic acid RNA interference construct targeting the RNA molecules expressed from the at least one gene involved in the TGS and/or PTGS mechanism;
- inserting in the plant at least one nucleic acid construct comprising a strong promoter coupled to a polynucleotide sequence comprising a coding sequence of a recombinant protein,
wherein (i) the coding sequence of the recombinant protein is linked in a reading frame to an oil body protein coding sequence; and/or (ii) the step of inactivating at least one gene includes the inactivation of at least one gene involved in the TGS mechanism and at least one gene involved in the PTGS mechanism,
wherein the step of inserting is performed by using a method selected among: CRISPR-Cas 9, TALENs, Meganuclease, Zinc finger nuclease with particle gun transformation and Agrobacterium tumefaciens, wherein the step of inactivating may be performed after the step of inserting, and
- optionally recovering the recombinant protein and the oil body protein and optionally purifying the recombinant protein recovered.

13. A method for obtaining a genetically engineered plant or part thereof as in claims 1 to 10, the method comprising:
- inactivating at least one gene involved in the TGS and/or PTGS mechanism in a plant or part thereof by :
(1) generating at least one genetic alteration in the coding sequence of one or more gene involved in the TGS and/or PTGS mechanism, such generating step being performed by using a method selected among: CRISPR-Cas 9, TALENs, Meganuclease, TILLING and Zinc finger nuclease, synthetic RNAi, physical or chemical mutagenesis and combinations thereof; and/or
(2) implementing at least one nucleic acid RNA interference construct targeting the RNA molecules expressed from the TGS and/or PTGS mechanism,
- inserting in the plant at least one nucleic acid construct comprising a strong promoter coupled to a nucleic acid sequence comprising a coding sequence of a recombinant protein,
wherein (i) the coding sequence of the recombinant protein is linked in a reading frame to an oil body protein coding sequence; and/or (ii) the step of inactivating at least one gene includes the inactivation of at least one gene involved in the TGS mechanism and at least one gene involved in the PTGS mechanism,
wherein the step of inserting is performed by using a method selected among: CRISPR-Cas 9, TALENs, Meganuclease, Zinc finger nuclease with particle gun transformation and Agrobacterium tumefaciens, and
wherein the step of inactivating may be performed after the step of inserting.

14. A method as in claim 13, wherein the method further comprises :
- collecting the seeds of the genetically engineered plant with at least one inactivated gene involved in the TGS and/or PTGS mechanism and the at least one nucleic acid construct inserted;
- growing new plants from the seeds and/ or plant tissues ;
- selecting the heterozygotes or homozygotes new-grown plants which have at least one inactivated gene involved in the TGS and/or PTGS mechanism and the nucleic acid construct.

15. A fusion protein comprising a recombinant protein and an oil-body protein produced by, or obtained from
(i) the genetically engineered plant or part thereof according to any one of claims 1 to 10, or
(ii) the genetically engineered plant or part thereof obtained from the method of claim 13, or
(iii) the heterozygotes or homozygotes new-grown plants selected of claim 14.


SEQUENCE LISTING

<110>  Core Biogenesis

<120>  TITLE OF THE INVENTION: A GENETICALLY ENGINEERED PLANT OR PART
       THEREOF ADAPTED FOR THE PRODUCTION OF RECOMBINANT PROTEINS AND
       PROCESS THEREOF

<130>  072797 EP

<160>  11

<170>  PatentIn version 3.5

<210>  1
<211>  3402
<212>  DNA
<213>  Arabidopsis thaliana

<400>  1
atggtgtcag agacgacgac gaaccgatca acggttaaaa tctcaaacgt tcctcaaacc      60

atagtcgccg acgaactcct ccggttctta gaactccacc tcggcgaaga tactgtattc     120

gcactcgaaa tcccaacaac tcgtgacaat tggaagccaa gagacttcgc tcgagtacaa     180

ttcactaccc tcgaagtcaa atctcgagcc cagcttctct cttctcaaag caagctcctt     240

ttcaaaaccc ataacctaag actctctgaa gcttatgatg atatcatccc tcgtcctgta     300

gatccaagga aaaggctaga cgacatcgtt ttgactgttg ggtttcctga gtctgatgaa     360

aaaaggtttt gtgccctaga aaaatgggat ggtgtgaggt gttggatttt gactgagaag     420

agaagagttg agttttgggt ttgggaaagt ggtgattgtt ataagattga agttaggttt     480

gaggatatta ttgaaactct tagttgttgt gttaatggtg acgcttctga gattgatgca     540

tttcttctca agctgaaata tggaccgaag gtatttaaaa gagtaacagt tcacatagcc     600

acaaagttta agtctgatcg gtacagattc tgcaaggagg attttgattt catgtggatt     660

cggacaactg atttctctgg ttcgaaatca attggtacat caacttgctt ttgtttggaa     720

gtccacaatg gctcaacgat gttagacatt ttctcgggct taccttacta ccgagaagac     780

actttgagtc taacttatgt ggatgggaag acctttgctt ctgcagctca aattgttccc     840

ctcttgaatg ccgccatttt ggggttagag tttccatatg agatcctttt ccaactcaat     900

gcgcttgttc atgcgcagaa aatcagtctt tttgctgcgt cagatatgga actgatcaaa     960

atccttcgtg gtatgagttt ggaaaccgca ttggtgatcc tcaagaaact tcaccagcaa    1020

agttctatat gttatgatcc tgtcttcttt gtcaagactc agatgcaatc tgtggttaag    1080

aagatgaagc attcccctgc atcagcttat aaaagattga ccgagcagaa catcatgagt    1140

tgtcagagag cttacgttac accctcaaag atctatctgt tgggtccaga gcttgagact    1200

gcaaattatg ttgtgaagaa ctttgcagag catgtctcgg atttcatgag agttactttt    1260

gtggaagaag attggagcaa gcttcccgca aatgctctct ctgtgaactc caaggaaggc    1320

tattttgtga agccctctag aaccaacatt tataacaggg tgttgtcaat ccttggagaa 1380

gggattaccg ttgggcctaa gaggtttgag ttcttggctt tttcagcgag ccaactgcga 1440

ggaaattcgg tctggatgtt tgcttctaac gagaaagtaa aagcggaaga tataagagaa 1500

tggatgggtt gtttccgtaa aatccgaagc atttccaaat gtgctgctag gatgggtcag 1560

ttgttcagtg cttcgcggca aacacttatt gtccgtgcac aagatgtgga gcagattccc 1620

gacatcgaag tgacaactga tggtgctgat tactgcttct cggatggcat tgggaaaatt 1680

tcacttgcat ttgccaagca agttgcacag aagtgtggat tgagtcatgt cccttctgcc 1740

tttcaaattc gatacggtgg ctacaaaggt gtgattgctg ttgaccgcag ttccttccga 1800

aagttgtctc tgcgtgatag tatgcttaaa tttgactcga acaacaggat gctgaacgtt 1860

accaggtgga cagagtcgat gccttgcttc ttaaaccggg agatcatttg ccttttgtcg 1920

acccttggaa tagaagatgc aatgtttgag gcgatgcaag cggtgcattt atctatgctt 1980

gggaatatgc ttgaagaccg cgatgcagca cttaatgttc tgcagaaatt gagcggagaa 2040

aattccaaga atttgctagt taagatgctg cttcaaggat atgcaccgag ttcagaacct 2100

tacctctcaa tgatgcttcg tgtgcaccac gagagccagc tttctgaact aaagagcaga 2160

tgcaggatac ttgtaccgaa aggacggatc ttgatcggtt gcatggatga aatgggtatc 2220

ctggagtatg gccaagtgta tgttcgtgta accctgacta aagcggaact gaaatctcgc 2280

gatcagagct actttcgcaa gattgatgag gaaacatctg tggttattgg gaaagtggtc 2340

gtgacgaaaa acccatgtct tcaccctgga gatattagag ttcttgatgc tatatatgag 2400

gtccatttcg aagaaaaggg atatctcgac tgcatcatct ttcctcagaa gggagaaaga 2460

ccacatccaa atgaatgttc tggtggcgat ctcgacggag accagttttt tgttagctgg 2520

gatgagaaga ttatacctag cgaaatggac cctccaatgg actatgctgg aagcaggcct 2580

cgtctaatgg atcatgacgt cacgttagag gaaatccaca aattttttgt ggattatatg 2640

ataagcgaca cgcttggggt gatctcaact gcacatttgg ttcacgcaga ccgtgaccct 2700

gaaaaagccc ggagccagaa gtgtctagag ttagcaaatc ttcactctag ggctgttgat 2760

tttgcaaaaa ctggagctcc agctgagatg ccttatgcct taaagcccag agagttccct 2820

gattttctgg aacggtttga gaaaccaaca tacatctctg agtctgtgtt tgggaaacta 2880

taccgtgctg tgaagagctc tctagcacag agaaagccag aagctgagag cgaggacacg 2940

gtagcttatg atgtgacact tgaagaagct ggctttgaga gctttataga gacagcgaaa 3000

gcccatagag acatgtatgg tgagaaactg acctcgttga tgatatacta tggagctgct 3060

aacgaagagg agattctcac aggcattttg aaaaccaagg agatgtatct ggcgagagat 3120

aaccggaggt atggtgatat gaaggataga attacgcttt ccgtgaaaga tttgcataaa 3180

gaggccatgg gatggttcga gaaaagctgc gaggacgaac aacagaagaa gaagctagca 3240

tcggcgtggt actatgtaac atacaatcca aaccatcgcg atgagaagtt gacattcttg 3300

agtttcccat ggatcgtagg tgacgttttg cttgatataa aggctgaaaa tgcacagaga 3360

cagagcgttg aagagaaaac aagtggactt gtatccattt ga 3402

<210> 2
<211> 3628
<212> DNA
<213> Camelina sativa

<400> 2
ttttctgatt ctctgggaat cactcttctt cttcactccc tctctctctc tctctctctc 60

cgttcacttg ctcttggggt caatcatggt gtcagaggcg acgacggcta cgaaccgacc 120

aaccgttaaa gtctcaaatg ttcctcaaac catagtcgcc gacgaactcc tccggttcct 180

cgagctccac ctcggcgaag acacagtgta cgctctcgaa atcccaacaa ctcgcgagaa 240

ttggaagcca cgggacttcg ctagagtaca attcagtacc ctcgaagtca aatctcgagc 300

ccagcttctc tcttcacaaa gcaagctcct tttcaaaacc cataacctaa gactctccga 360

agcttacgat gacatcatcc ctcgccctgt agatccacgg agaaggctag aagacgtcgt 420

tttgactgtt gggttccctg agtctggtga agagaggttc tgtgccctag aaaaatgggg 480

aggcgtgagg tgttggattc tggaagagaa gaggagactt gagttttggg tttgggaatg 540

tggtgaatgt tataagcttg aagttaggtt tgaggatatt gttgaaactc ttggctgctg 600

tgttaatggt gacgcttctg agattaatgc gtttcttctc aagctgaaat atggaccaaa 660

ggtttttaaa agagtgacag gacctcacat agccacaaag tttaaatctg atcggtaccg 720

tttctgcaag gaggattttg atttcatgtg ggttcgcaca actgatttct ctggttcaaa 780

gtcaatcggt acatcaactt gcttttgttt ggaggtagag aatggctcca agatgttaga 840

cattttctcg tgtttgcctt actacagaga agacactttg agtctaacca ctgtggatgg 900

gactgcgttt gcctctgcat gtcaaattgt tcccctcttg aatgctgcca gtctggggtt 960

agagtttcca tatgagatcc tctttcaact caattcgctg gttcacaatc agaaaatcag 1020

tctttttgct gcatcaaata tggaactgat caacatcctt cgtggtttga gcttggaaac 1080

cgcattggtt atcctcaaga agcttcacca gcaaagctct atatgttatg atcctgtctc 1140

ctttgtcaag actctgatgc aatccccggt taagaagatg aagcattccc ctgcatcagc 1200

taatacaaga ctgaccgagc agaatatcat gagttgtcag agagcctacg ttacaccctc 1260

aaagatttat ctgttgggtc cagagcttga gaaagctaac tatgttgtga agaactttgc 1320

agagcatgtc tcagatttca tgagaattac ttttgtggaa gaagattgga gcaagattcc 1380

agcaaatgct ctttctgtga actccaagga aggctatttt gtgaagccct ttagaaccaa 1440

catttataac agggtgttgt caatccttgg agaagggatc actgttgggc ctaagaggtt 1500

tgagttcttg gctttttcag ccagccaact gcgaggaaac tcagtctgga tgtttgcttc 1560

taacgagaaa gttaaagcag aagatataag agaatggatg ggttgtttcc gtaaaatccg 1620

aagcatctcc aaatgtgctg ctaggatggg tcagttgttc agtgcttcac ggcagacagt 1680

tactgtccgt gcacaagatg tggagcagat tcctgacatt gaagtgacta ctgatggtgc 1740

tgattattgc ttctcggatg gcatcgggaa aatttcgttg gcatttgcta ggcaagttgc 1800

tcagaagtgt gggcttgatt atgtcccttc tgcatttcaa attcgatacg gtggctacaa 1860

aggtgtgatc gccgttgacc gcagttcctt cagaaagatg tctctgcgtg atagtatgct 1920

aaagtttgac tcaaacaaca ggatgcttaa cgtaaccagg tggacagagt cgatgccttg 1980

cttcttaaac cgggagatca tttgtctttt gtcgactctt ggaatagaag atgcagtgtt 2040

tgaggccatg caagcgatgc atttatctat gctggggaat atgcttgaag accgcaatgc 2100

agcacttaat gtgctgcaga aattgagcgg agaaaattcc aagaatctgc tagttaagat 2160

gctgctacaa ggatacgcac cgagttcaga accgtacctc tcaatgatgc ttcgtgtgca 2220

ccacgagagc caactttctg aactaaagag taggtgcagg atacttgtaa cgaaaggacg 2280

gatcttgatc ggttgcatgg atgaaatggg tattctggag tatggccaag tgtatgttcg 2340

tgtaaccctg actgaagcgg aactgaaatc tcgtgaacag agttacttcc gcaagattaa 2400

tgaggaaaca tctgtggtga ttgggaaagt ggtcgtgacg aaaaacccat gtcttcaccc 2460

tggagatatc agagttcttg acgctatata tgaggtcagt ttcgaagaaa agggatttgt 2520

tgactgcatc atctttcctc agaaggagaa aaggccacat cccaatgaat gttctggtgg 2580

tgatctcgac ggagaccagt tttttgttag ttgggatgag aagcttatac cgagccaaat 2640

ggaccctcca atggactacg ctggaagcag acctcgtcta atggatcatg acgttaccct 2700

agaggaaata cacaaatttt ttgtggatta tatgataagc gacacgctgg gagtgatctc 2760

aactgcacat ttggttcatg cagaccgtga ccctgaaaaa gccaggagcc agaagtgtct 2820

agagttagca catcttcact ctaggctgtt tgattttgcg aaaactggag ctccagcgga 2880

gatgccttat gccttaaagc ccagagagtt ccctgatttc ctggaacggt tcgagaaacc 2940

aacatacatc tctgagtctg tgtttgggaa actttaccgt gctgtgaaaa gctcgttagc 3000

acagaggaag ccagaagctg agagcgagga cacgatagct tatgattcca cacttgaaga 3060

agctggctat gagagcttta tagagacagc caaagcccac agagacatgt atgctgagaa 3120

actgacctcg ttgatgatat actacggagc tgtaaacgaa gaagagatcc tcacaggcat 3180

tttgaaaact aaggagatgt atctggcgag agataaccgg aggtatggtg atatgaagga 3240

tagaatcacc ttgtctgtga aggatttgca gagagaggcc ttgggatggt tcgataaaag 3300

ctgcgaggag aaacaagaga agaagaagct agcttcagct tggtattacg taacatacaa 3360

cccgagccat gtcgatgaga agttgaaatt cctgagtttt ccgtggatcg ttggtgacat 3420

attgctagag attaaggctg aaaatgcaca gagtcagagc gttgaagacc aaacaaatgg 3480

acttgtatcc atttgattac cctatcccta tctatctatc actcgttgct gtagtataat 3540

tgctagatta agacaattga gaccagaaca gaacgtcgct tgtatatgcg tcactcaaaa 3600

ttatcaaata ctaatgacta attaatta 3628

<210> 3
<211> 3377
<212> DNA
<213> Camelina sativa

<400> 3
caacggttaa agtctccaat gttcctcaaa ccatagtcgc cgacgaactc ctccgcttcc 60

tcgagcttca cctcggcgaa gacacagtgt tcgctctcga aatcccaaca actcgcgaga 120

attggaagcc acgggacttc gctcgagtac aattcagtac cctcgaagtc aaatctcgag 180

cccagcttct ctcttcacaa agcaagctcc ttttcaaaac ccataaccta agactctccg 240

aagcttacga tgacatcata cctcgccctg tagatccacg gagaaggcta gaagacgtcg 300

ttttgactgt tgggtttcct gagtctggtg aagagaggtt ctgtgcccta gaaaaatggg 360

gaggcgtgag gtgttggatt ctggaagaga agaggagact tgagttttgg gtttgggaat 420

gtggtgaatg ttataagatt gaagttaggt ttgaggatat tgttgaaact cttggctgcg 480

ttgttaatgg tgacgcctct gagattaatg cgtttcttct gaggctgaaa tatggaccaa 540

aggtatttaa aagagtaaca ggacctcaca tagccgcaaa gtttaaatct gatcggtacc 600

gtttctgcaa ggatgatttt gatttcatgt gggttcgcac aactgatttc tctggttcaa 660

agtcaatcgg tacatcaact tgcttttttt tggaggtaga gaatggctcc aagatgttag 720

acattttctc gtggttgcct tactacagag aaggcacttt gagtctaacc actgtggatg 780

ggaaagcgtt tgcctcttca tgtcaaattg ttcccctctt gaatgctgcc aatctggggt 840

tagagtttcc atatgagatc cttttccaac tcaattcgct ggttcacaat aaaaaaatca 900

gtctttttgc tgcatcaaat atggaactga tcaacatcct tcgtgggttg agattggaaa 960

ccgcattggt tatcctcaag aaacttcacc agcaaagctc tatatgttat gatcctgtct 1020

cctttgtcaa gactcagatg caatccccgg ttaagaagat gaagcattcc cctgcatcag 1080

ctaatacaag actgaccgag cagaatatca tgagttgtca gagagcctac gttacaccct 1140

caaagattta tctgttgggt ccagagcttg agaaagctaa ctatgttgtg aagaactttg 1200

cagagcatgt ctcagatttc atgagaatta cttttgtgga agaagattgg agcaagattc 1260

cagcaaatgc tctttctgtg aactccaagg aaggctattt tgtgaagccc tttagaacca 1320

acatttataa cagggtgttg tcaatccttg gagaagggat cactgttggg cctaagaggt 1380

ttgagttctt ggctttttca gccagccaac tgcgaggaaa ctcagtctgg atgtttgctt 1440

ctaacgagaa agttaaagca gaagatataa gagaatggat gggttgtttc cgtaaaatcc 1500

gaagcatctc caaatgtgct gctaggatgg gtcagttgtt cagtgcttca cggcagacag 1560

ttactgtccg tgcacaagat gtggagcaga ttcctgacat tgaagtgact actgatggtg 1620

ctgattactg cttctcggat ggcattggga aaatttcatt ggcatttgct aggcaagttg 1680

ctcagaagtg tgggcttgat tatgtccctt ctgcatttca aattcgatac ggtggctaca 1740

aaggtgtgat cgccgttgac cgcagttcct tcagaaagat gtctctgcgt gatagtatgc 1800

taaagtttga ctcaaacaac aggatgctta acgtaaccag gtggacagag tcgatgcctt 1860

gcttcctaaa ccgggagatc atttgccttt tgtcgactct tggaatagaa gatgaagtgt 1920

ttgaggcgat gcaagcgttg catttatcta tgctggggaa catgcttgaa gaccgcaatg 1980

cagcacttaa tgtgctgcag aaattgagcg gagaaaattc caagaatctg ctagttaaga 2040

tgctgctaca aggatacgca ccgagctcag aaccgtacct ctcaatgatg cttcgtgtac 2100

accacgagag ccagctttct gaactaaaga gtaggtgcag gatacttgta ccgaaaggac 2160

ggatcttgat cggttgcatg gatgaaatgg gtatcctgga gtatggccaa gtgtatgttc 2220

gtgtaaccct gactgaagcg gaactgaaat ctcgtgaaca gagttacttc cgcaaggtta 2280

atgaggaaac atctgtggtg attgggaaag tagtcgttac gaaaaaccca tgtcttcacc 2340

ctggagatat cagagttctt gacgctatat atgaggtcag tttcgaagaa aagggatttg 2400

tcgactgcat catctttcct cagaagggag aaaggccaca tcccaatgaa tgttctggtg 2460

gtgatctcga cggagaccag ttttttgtta gttgggatga gaagcttata ccaagccaaa 2520

tggaccctcc aatggactac gctgctagca gacctcgtat aatggatcat gatgtcaccc 2580

tagaggaaat ccacaaattt tttgtggact atatgataag cgacacgctg ggagtgatct 2640

caactgcaca tttggttcat gcagaccgcg accctgaaaa agccaggagc cagaaatgtc 2700

tagagttagc gcatcttcac tctagggctg ttgattttgc caaaactgga gctccagcgg 2760

agatgcctta cgccttaaag cccagagagt tccctgattt cctggaacgg ttcgagaaac 2820

caacatacat ctctgagtct gtgtttggga aactttaccg tgctgtgaaa agctcgttag 2880

cacaaaggaa gccagaagct gagagcgagg acacgatagc ttatgattcc acacttgaag 2940

aagctggcta tgagagcttt atagagacag ccaaaaccca cagagacatg tatgctgaga 3000

aactgacctc gttgatgata tactacggag ctgtaaacga agaagagatc ctcacaggca 3060

ttttgaaaac taaggagatg tatctggcga gagataaccg gaggtatggt gatatgaagg 3120

atagaatcac cttgtctgtg aaggatttgc agagagaggc cttgggatgg ttcgagaaaa 3180

gctgcgagga gaaacaagag aagaagaagc tagcctcggc ttggtactac gtaacataca 3240

acccgagcca ggtcgatgag aagttgaaat tcctgagttt tccgtggatc gttggtgaca 3300

ttttgctaga gattaaggct gaaaatgcac agagtcagag cggtgaagac caaacaaatg 3360

gacttgtatc cgtttga 3377

<210> 4
<211> 3111
<212> DNA
<213> Camelina sativa

<400> 4
cctgtagatc cacggagaag gctagaagac gtcgttttga ctgttgggtt ccctgagtct 60

ggtgaagaga ggttctgtgc cctagaaaaa tggggaggcg tgaggtgttg gattctggaa 120

gagaagagga gacttgagtt ttgggtttgg gaatgtggtg aatgttataa gcttgaagtt 180

aggtttgagg atattgttga aactcttggc tgctgtgtta atggtgacgc ctctgagatt 240

aatgcgtttc ttctcaagct gaaatatgga tcgaaggttt ttaaaagagt gacaggacct 300

cacatagcca caaagtttaa atctgatcgg taccgtttct gcaaggagga ttttgatttc 360

atgtgggttc gcacaactga tttctctggt tcaaagtcaa tcggtatatc aacttgcttt 420

tgtttggagg tagagaatgg ctcaaagatg ttagacattt tctcaagctt gccttactac 480

agagaagaca cgttgagtct aaccactgtg gatgggaaag tgtttgcctc tgcatgtcaa 540

attgttcccc tcttgaatgc tgccaatctt gggttagagt ttccatatga gatcctttac 600

caactcaatt cgcttgttca caatcagaaa atcagtattt ttgctgcgtc aaatatggaa 660

ctgattaaca tccttcgtgg tttgagcttg gaaaccgcat tggtgatcct caagaaactt 720

caccagcaaa gctctatatg ttatgatcct gtctcctttg tcaagactca gatgcaatcc 780

ccggttaaga agatgaagca ttcccctgca tcagctaata caagattgac cgagcagaat 840

atcatgagtt gtcagagagc ctacgttaca ccctcaaaga tttatctgtt gggtccagag 900

cttgagaaag ctaactatgt tgtgaagaac tttgcagagc atgtctcaga tttcatgaga 960

attacttttg tggaagaaga ttggagcaag attccagcaa atgctttttc tgtgaactcc 1020

aaggaaggct attttgtgaa gccctttaga accaacattt ataacagggt gttgtcaatc 1080

cttggagaag ggatcactgt tgggcctaag aggtttgagt tcttggcttt ttcagccagc 1140

caactgcgag gaaactcagt atggatgttt gcgtctaacg agaaagttaa agcagaagat 1200

ataagagaat ggatgggttg tttccgtaaa atccgaagca tatcaaaatg tgctgctcgg 1260

atgggtcagt tgttcagtgc ttcacggcag acagttactg tccgtgcaca agatgtggag 1320

cagattcctg acattgaagt gactactgat ggtgctgatt actgcttctc ggatggcata 1380

gggaaaattt cattggcatt tgctaagcaa gttgctcaga agtgtggact ggattatgtc 1440

ccttctgcat ttcaaattcg atacggtggc tacaaaggtg tgatcgccgt tgaccgcagt 1500

tccttcagaa agatgtctct gcgtgatagt atgctaaagt ttgactcaaa caacaggatg 1560

cttaacgtaa ccaggtggac agagtcgatg ccttgcttct taaaccgaga gatcatttgc 1620

cttttgtcga ctcttggaat tgaagatgaa gtgtttgagg cgatgcaagc tatgcattta 1680

tctatgctag ggaatatgct tgaagaccgc aatgcagcac ttaatgtgct gcagaaattg 1740

agcggagaaa attccaagaa tctgctagtt aagatgctgc tacaaggata tgcaccgagt 1800

tcagaaccgt acctctcaat gatgcttcgt gtacaccacg agagccaact ttctgaacta 1860

aagagtaggt gcaggatact tgtaccgaaa ggacggatct tgatcggttg catggatgaa 1920

atgggtatcc tggattatgg ccaagtgtat gttcgtgtaa ccctgactga agcggaactg 1980

aaatctcgtg aacagagcta cttccacaag attaatgagg aaacatctgt ggtgattggg 2040

aaagtggtcg ttacgaaaaa cccatgtctt caccctggag atatcagagt acttgacgct 2100

atatatgagg tcagtttcga agaaaaggga tttgtcgact gcatcatctt tcctcagaag 2160

ggagaaaggc cacatccaaa tgaatgttct ggtggtgatc tcgacggaga ccagtttttt 2220

gttagttggg atgagaagct tataccaaac caaatggacc ctccaatgga ctacgctgca 2280

agcagacctc gtctaatgga tcatgacgtt accctagagg aaatacacaa tttttttgtg 2340

gattatatga taagcgacgc gcttgggatg atctcaactg cacatttggt tcatgcagac 2400

cgtgacccag aaaaagccag gagccagaag tgtctagagc tagcacatct tcactctagg 2460

gctgttgatt ttgccaaaac tggagctccg gctgagatgc cttatgcctt aaagcctaga 2520

gagttccctg atttcttgga acggttcgag aaaccaacat acatctctga gtctgtgttt 2580

gggaaacttt accgtgctgt gaaaagctcg ttagcacaga gaaagccaga agctgagagc 2640

gaggacacga tagcttatga ttcgacactt gaagaagctg gctttgagag ctttatagag 2700

acagccaaag cccatagaga catgtatgct gagaaactga cctcgttgat gatatactac 2760

ggagctgtta acgaagaaga gatcctcaca ggcattttga aaaccaagga aatgtatctg 2820

gctagggata accggaggta tggtgatatg aaggatagaa tcacgttgtc tgtgaaggat 2880

ttgcagagag aggccatggg atggttcgac aaaagctgcg tggagaaaca agagaagaag 2940

aagctagctt cggcttggta ctacgtaaca tacagcccga gccaggtcga tgagaagttg 3000

agattcttga gttttccgtg gatcgttggt gacattttgc tagagatcaa ggctgaaaat 3060

gcacagagac agagcgttga agaccaaaca agtggacttg tatccatttg a 3111

<210> 5
<211> 3591
<212> DNA
<213> Arabidopsis thaliana

<400> 5
atggggtcag agggaaatat gaagaagtcg gttgtgacac aagtaagcat tggtggattt 60

ggggaatcca ctactgcaaa acaactcaca gattaccttg aagatgaagt aggaattgtt 120

tggcgttgca gattaaaaac ttcgtggact cctcctgggt cttatcctaa ttttgaaatc 180

gcagacactt caaatatccc cagtattgat gaatataaga aagtggagcc tcatgccttt 240

gtccatttcg cagtttttga atcagctggt cgtgccatgg atgctgcagg gcaatgcaac 300

ctcatcttgg atggccagcc gttaaaagtc agtttgggtc ctaagaatcc atattccctt 360

aatcaaagaa gaagaacgac tgtaccttat aagttggctg gtattacact tgagattggg 420

acattggttt ctcgggatga tttttttgtt tcttggagag ctgaaggggt tgatttcctg 480

gtggatcctt ttgacaacac gtgcaagttt tgtttcagaa agagcactgc cttctctttc 540

aaggatgcag tgatgcatgc tgtgatcaat tgtgattata agttggagtt attggtgaga 600

gatatacaaa cagtcaggca gtataaaacc ttgcatggct ttgtgcttat tttgcagctg 660

gcttcttcac cccgtgtctg gtacagaact gctgatgatg atatttacga tactgtccct 720

ggcgatctct tggatgatga tgatccttgg atccgtacca ctgattttac tcaagttggg 780

gcaattggtc gatgtcattc atatcgagtg ctcatatctc cgcggtatga aaataaactg 840

agaacagcct tagattattt taggatgcgg agggtgcaag aggaacgtgt gaggtggcct 900

ccccgtatcc gtaatgagcc ttgttttggg gagcctgtgt cagatcattt cttctgcatt 960

catcacaagg aaggaatctc ttttgagatc atgtttctag taaattcggt attgcacagg 1020

ggtgtcttta accagtttca gttgactgag cgattctttg atcttctaag aaaccaaccc 1080

aaggacgtca atatagcttc tctcaagcat ctctgtacct ataaacgacc agtttttgat 1140

gcgtacaaga ggttgaagct tgttcaggaa tggattcaga aaaatccaaa gcttttaggg 1200

agtcatgaac aatctgagga tatctctgag atcagaagac tagtaattac cccaaccaga 1260

gcctattgcc tacccccaga agttgagctc tccaacaggg tactcaggag atacaaagct 1320

gttgctgaaa gatttttgcg ggtaactttc atggatgaaa gtatgcagac cataaattcg 1380

aatgttctct cttactttgt tgctcctatt gtgaaggatt tgacatcaag ttctttctcc 1440

cagaagacct acgtttttaa aagagtgaag agcatattaa ccgatgggtt taaactatgt 1500

ggtagaaaat acagttttct agcattctca gccaatcaac tgagagaccg ctctgcatgg 1560

ttctttgctg aagacggaaa aacacgtgtg tcagatataa aaacatggat ggggaagttc 1620

aaagacaaga atgtggcaaa atgtgctgct aggatgggcc tgtgcttctc ctccacatat 1680

gccactgtag atgtcatgcc tcacgaggtt gacactgagg ttccagatat tgagagaaat 1740

gggtatgttt tctctgacgg aattggtaca atcacacctg acctcgctga cgaagtaatg 1800

gagaaactta agttggatgt gcactacagc ccttgtgctt atcagatacg ttacgcaggt 1860

ttcaaagggg ttgttgctcg ttggccatca aaaagtgatg gaatcaggct agcccttcga 1920

gacagtatga agaagttctt ttccaaacat acgatcttgg agatctgttc ctggacgagg 1980

tttcaacctg ggttcttaaa tcggcagata attacccttc tatccgtact aggtgttccg 2040

gatgaaatat tctgggatat gcaggaatcc atgctctata aactgaaccg catccttgat 2100

gatacagatg tggcatttga agttctcacg gcatcatgtg ctgaacaggg aaacactgca 2160

gctatcatgc ttagtgcagg tttcaaacca aaaaccgagc cgcatctacg cgggatgttg 2220

tcttcagtca gaattgcaca actctggggt ctcagagaaa aatctcgtat ttttgttact 2280

tcaggaaggt ggctaatggg ttgcctagac gaagcaggga tacttgaaca tggccaatgc 2340

tttattcaag tctctaaacc gtctatagaa aattgtttct ccaaacatgg gtctcgtttt 2400

aaggagacaa agacagatct ggaagtagtt aaaggctatg tagccattgc taagaatcct 2460

tgtcttcacc caggggatgt aaggatttta gaagctgttg atgtacccca gctgcatcac 2520

atgtatgact gccttatttt ccctcagaaa ggtgataggc cgcatacaaa cgaagcttct 2580

ggcagtgacc ttgacgggga cctgtacttt gtggcttggg atcagaaact catccctccc 2640

aacaggaaaa gctatccggc catgcattat gatgcagctg aagagaagag tttaggccgt 2700

gctgtcaacc accaggacat aatcgatttc tttgcaagaa acttggcgaa tgagcagttg 2760

ggcacaattt gcaatgcaca cgtcgttcat gctgatagaa gtgagtatgg agccatggac 2820

gaagaatgtt tgctactggc agaactagct gccactgcag ttgatttccc aaagacaggg 2880

aaaattgtgt caatgccctt ccacctaaaa ccaaaactct acccagattt catgggaaaa 2940

gaagactacc aaacttacaa gtcgaacaaa atcttgggtc ggctttacag acgggtaaaa 3000

gaggtttatg atgaagatgc agaagcttcc tcagaagaaa gcacagaccc aagtgccatc 3060

ccttatgacg ctgttcttga aataccggga tttgaagatt tgatccctga ggcatggggt 3120

cacaaatgtt tgtacgacgg gcaactcatt ggtcttcttg ggcaatacaa ggtgcagaaa 3180

gaggaagaga ttgtgacggg tcacatctgg tccatgccca aatacacaag caagaaacaa 3240

ggcgaactga aagaaagact gaagcattct tataattccc ttaaaaagga gttcaggaaa 3300

gtatttgagg aaacaatccc tgaccatgaa aatctcagcg aggaggaaaa aaacatcttg 3360

tatgagaaga aggcttcagc ttggtatcat gtaacttacc atcccgagtg ggtgaagaag 3420

tctttagagc tgcaagatcc agatgagtcc agtcatgcgg cgatgctgag ttttgcttgg 3480

attgcagctg attatcttgc aagaatcaaa atccggtcac gggaaatggg aagtatcgac 3540

tcagccaagc ctgttgattc tttggctaag tttcttgctc agcgtctcta a 3591

<210> 6
<211> 3965
<212> DNA
<213> Camelina sativa

<400> 6
cgtgggagga agtcaagaat aataatgacg catggtttca ctttcagcta tggcgatttc 60

tccttcctaa tttagcctct actcacttac taatcttctc tccggtctcc gcgttgctat 120

ctgtttcgtc ttcagtatcg atttcgttca ctgtggcttc acggagaatg gggtcagagg 180

gaaacatgaa gaactcggtt gtgacacaag taagcattgg tggattcggt gagtccacta 240

ctgcaaaaca acttactgat tacctcgaag acgaagtagg acttgtttgg cgatgcagat 300

taaagaactc ttggactccg cctgggtctt atcctaattt cgaaatcgca gacacttcaa 360

aaatccccaa aatcgatgat tacaagaaag tggagcctca tgcgtttgtc catttcgcac 420

ttcctgaatc aacagcacgt gctatggatg ctgcagggca atgcaagctc atcctcgatg 480

gccagccttt aaaagtcagc ttggggccta agaacccata tacacttaac cagagaagaa 540

gaacgactac accttataag ttgactggtg tttcgatcga gattgggact ttggtttccc 600

gggatgagtt tcttgtttct tggagagctg aaggtgttga tttccttgta gatccttttg 660

acaacacttg caagttttgc ttcaggaaga gcactgcgtt ctctttcaag gacgcagtga 720

tgcatgctgt gataaattgt gattataagt tggagttgtt ggtgagagac ataagaacgg 780

ttaggcagta taaggatttg catggctttg tgcttctttt gcagctggct tcgtcacccc 840

gtgtctggta cagaactgcg gatgatgaca tttatgagac tgttcctttc gatctcttgg 900

atgatgatga cccttggatc cgtaccactg atttcactca agttggggcg attggtcgat 960

gtctttcgta tagagtgctc atatctcctc ggtatgagaa gaaattgaca acagctttag 1020

attatctaag gatgcggagg gtgcaagagg aacgtgtgag gtggcctcct cggatccgtg 1080

atgagccttg ttttggggag cctgtgtcgg atcatttctt ctgcattcat cacaaggaag 1140

gaatctcctt tgagatcatg tttctagtga actcggtatt gcacagaggg gtttttaacc 1200

agtttcagtt gactgagcgt ttctttgatc ttctcagaaa ccaacccaga gatgtcaata 1260

tagcttctct caagcatctc tgtacctata aaaaaccagt ttttgatgcg tacaagaggt 1320

tgaagcttgt tcaggaatgg attcagaaaa atccaaagct tttagggagt catgaacaat 1380

ctgaggatat ctctgagatc agaaggttag tgattacccc aaccagagcc cattgcctac 1440

ccccagaagt tgagctctcc aacagggtac ttaggaaata caaagctgtt gctgaaagat 1500

ttttgcgcgt aactttcatg gatgaaagta tgcagaccat aaattcgaat gttctctctt 1560

actttgttgc tcctattgtg aaggatctga catcaagctc tttctctcag aagacttacg 1620

ttttcaaaag agtgaagagc atattaaccg atgggtttaa actatgtggt agaaaatata 1680

gttttctagc attctcagcc aatcaactga gagaccgctc tgcttggttc tttgctgaag 1740

acgggaaaac aagagtgtca gatataaaaa catggatggg gaagtttaaa gacaagaatg 1800

tggcaaaatg tgctgctagg atgggactgt gcttctcctc cacatatgcc actgtagatg 1860

tcatgcttca cgaggttgac accgagcttc cagacattga gagaaatggg tatgttttct 1920

ctgatggaat tggtacaatc acacctgacc tcgctgacga agtaatggag aaacttaggt 1980

tggatgtgca ctacacccct tgtgcttatc agatacgtta cgcaggtttc aaaggagttg 2040

ttgctcgttg gccatcaaaa ggtgatggaa tccggctagc ccttcgagac agtatgaaga 2100

agttcaattc caaacatact atcttggaga tctgttcctg gacgaggttt caacctggat 2160

tcttaaatcg gcagataatt actctccttt ctgtactagg tgttccggat gaaatattct 2220

gggatatgca ggaatccatg ctctgtaaac tgaaccgcat ccttgatgat acagatgttg 2280

catttgaagt tctcacagca tcatgtgctg aacagggaaa cactgcagct atcatgctaa 2340

gtgcaggttt caaaccaaaa actgagccac atctacgtgg aatgttgtct tcagtcagaa 2400

ttgcacaact ctggggtctc agagaaaaat cacgtatttt tgttacttca ggaaggtggc 2460

taatgggttg ccttgacgaa gcagggatac ttgaacaggg ccaatgcttt atccaagtct 2520

caaaaccgtc tatagaaaat tgtttctcca aacatgggtc tcgttttaag gagacaaaga 2580

cagatctgca agtagttaaa ggctatgtag ccattgccaa gaatccttgt cttcacccag 2640

gggatgtaag gattttagaa gctgttgatg taccccagct gcatcacatg tatgactgcc 2700

ttattttccc tcagaaaggt gataggcccc atacaaacga agcttctggc agtgaccttg 2760

acggggacct gtactttgtg gcttgggatc agaaactcat ccctcccagc aaaaaaagct 2820

ttccggccat gcattatgat gcagctgaag agaagagttt aggccgtgct gtcaaccacc 2880

aggatataat agatttcttt gctagaaaca tggcaaatga gcatttgggt acaatttgca 2940

atgcacacgt cgttcatgct gatagaagtg agtatggagc catggacgaa gaatgtatgc 3000

tgctggcaga attagctgcc actgcagttg atttcccgaa gacggggaaa attgtgtcaa 3060

tgcccttcca cctaaaacca aaactctacc cagatttcat gggaaaagaa gactaccaaa 3120

cttacaagtc gaacaaaatc ttaggtcggc tttacagacg tgtgaaagaa gtgtatgatg 3180

aagatgcaga agcttcgtca gaagaaatcc cagatccaag tgacatccct tatgacactg 3240

atctcgagtt accgggattt gcagacttga tccccgaggc atggggtcac aaatgttctt 3300

acgacgggca gctcattggt cttcttgggc aatacaaggt ccagaaagag gaagagattg 3360

tgacgggcca tatctggtcg atgccgaaat acgctagtaa gaaacaaggg gaattgaaag 3420

aaagattgaa gcattcttat aactccctca aaaaggagtt cagaaaagta ttcgaggaaa 3480

caatcccgga ccaggaaaat ctcagcgatg aggaaaaaaa catcttgtat gagaagaagg 3540

cttcagcttg gtatcatgtc acttaccatc ccgagtgggt gaagaagtct ttagagctgc 3600

aagatccaga tgagtcgtct agtcatgcgg tgatgctgag ttttgcttgg attgcagctg 3660

attatcttgc aagaatcaaa atccggtcac gggaaatggg aaatatcgac tcagccaagc 3720

ctgttgattc tttggccaag tttcttgctc aacgtctcta aaaggtaatg ttcgaagccg 3780

taaggccact ctgccatgta tctgtggaaa ttcacagata ctcaatatat atatatataa 3840

gctagttttg aatgtactac tctggaaact ttgtgtgtga tgaattcagg atttaaacag 3900

ctcaactgtt tgccgttgtt ttgtggttat gtattctact aaaatttcat ttcaatttta 3960

ttaca 3965

<210> 7
<211> 3981
<212> DNA
<213> Camelina sativa

<400> 7
atgaagtaaa aaaaaaaaaa ggtttcactt tcagctatgg cgatttcgcc ttcctaattt 60

agcgcggcct actcactagt cttctctgcg gtctccgcgt tgcttctgtt tcgtttccag 120

tatcgatttc gttgattgag gacctcactg tagcagtgaa ttagggcttt tgcgaaaaat 180

ggggtcagag ggaaacatga agaactcggt tgtgacacaa gtaagcattg gtggatttgg 240

tgagtccact actgctaaac aacttacaga ttacctcgaa gacgaagtag gacttgtttg 300

gcgatgcaga ttaaagaact cttggactcc ccctgggtct taccctaatt tcgaaatcgc 360

agacacttca aaaatcccca aaatcgatga ttacaagaaa gtggagcctc atgcgtttgt 420

ccatttcgct attcccgaat caacagaacg tgccatggat gctgcagggc aatgcaagct 480

catcctcgat gggcagccgt taaaagtcag cttgggtcct aagaacccat atacacttaa 540

ccagagaaga agaacgacta caccttataa gttgactggt gtttcgattg agattgggac 600

tttggtttcc cgggatgagt ttcttgtttc atggagagct gaaggggttg atttccttgt 660

agatcctttt gacaacactt gcaagttttg cttcaggaag agcactgcgt tctctttcaa 720

ggacgcagtg atgcatgctg tgataaattg tgattataag ttggagttgt tggtgagaga 780

cataagaaca gtaaggcagt ataagaattt gcatggcttt gtgcttcttt tgcagctggc 840

ttcgtcaccc cgtgtctggt acagaactgc tgatgatgat atttacgaga ctgttccttt 900

tgatctcttg gatgatgatg acccttggat ccgtaccact gattttactc aagttggggc 960

gatcggtcga tgtctttctt atcgagtgct catatctcct cggtatgaga agaagttgac 1020

aacagcgtta gattatctaa ggatgcggag ggtgcaagag gaacgtgtga ggtggcctcc 1080

tcggatccgt gatgagccct cttttgggga gcctgtgtca gatcatttct tctgcattca 1140

tcacaaggaa ggaatctcct ttgagatcat gttcctagta aactcggtat tgcacagagg 1200

ggtttttaac cagtttcagt tgactgagcg tttctttgat cttctcagaa accaacccag 1260

agatgtcaat atagcttctc tcaagcatct ctgtacctat aaaaaaccag tttttgatgc 1320

gtacaagagg ttgaagcttg ttcaggaatg gattcagaaa aatccaaagc ttttagggag 1380

tcatgaacaa tctgaggata tctctgagat cagaaggtta gttattaccc caaccagagc 1440

ccattgccta cccccagaag ttgagctctc caacagggtt ctcaggaaat acaaagctgt 1500

tgctgaaaga tttttgcgcg taactttcat ggatgaaagt atgcagacta taaattctaa 1560

tgttctctct tactttgttg ctcctattgt gaaggatctg acatcaagct ctttctctca 1620

gaagacttac gttttcaaaa gagtgaagag catattaacc gatgggttta aactatgtgg 1680

tagaaaatat agttttctag cattctccgc caatcaactg agagaccgct ctgcatggtt 1740

ctttgctgaa gatgggaaaa caagagtgtc agatataaaa acatggatgg ggaagttcaa 1800

agacaagaat gtggcaaaat gtgctgctag gatgggactg tgcttctcct ccacatatgc 1860

cactgtagat gtcatgcttc acgaggttga caccgagctt ccagacattg agagaaatgg 1920

gtatgttttc tctgatggaa ttggtacaat cacacctgac ctcgctgacg aagtaatgga 1980

gaaacttagg ttggatgtgc actacacccc ttgtgcttat cagatacgtt acgcaggttt 2040

caaaggagtt gttgctcgtt ggccatcaaa aggtgatgga atccggctag ctcttcgaga 2100

cagtatgaag aagttcaatt ccaaacatac tatcttggag atctgttcct ggacgaggtt 2160

tcaacctgga ttcttaaatc ggcagataat tactctcctt tctgtactag gtgttccgga 2220

tgaaatattc tgggatatgc aggaatccat gctctgtaaa ctgaaccgca tccttgatga 2280

tacagatgtt gcatttgaag ttctcacagc atcatgtgct gaacagggaa acactgcagc 2340

tatcatgcta agtgcaggtt tcaaaccaaa aactgagcca catctacgtg gaatgttgtc 2400

ttcagtcaga attgcacaac tctggggtct cagagaaaaa tcacgtattt ttgttacttc 2460

aggaaggtgg ctaatgggtt gccttgacga agcagggata cttgaacagg gccaatgctt 2520

tatccaagtc tcaaaaccgt ctatagaaaa ttgtttctcc aaacatgggt ctcgttttaa 2580

ggagacaaag acagatctgc aagtagttaa aggctatgta gccattgcca agaatccttg 2640

tcttcaccca ggggatgtaa ggattttaga agctgttgat gtaccccagc tgcatcacat 2700

gtatgactgc cttattttcc ctcagaaagg tgataggccc catacaaacg aagcttctgg 2760

cagtgacctt gacggggact tgtactttgt ggcttgggat cagaaactca tccctcctag 2820

caaaaaaagc tttccggcca tgcattatga tgcagctgaa gagaagagtt taggccgtgc 2880

tgtcaaccac caggatataa tagatttctt tgcaagaaac atggcaaatg agcatttggg 2940

tacaatttgc aatgcacacg tcgttcatgc tgatagaagt gagtatggag ccatggacga 3000

agaatgtatg ctactggcag aattagctgc cactgcagtt gatttcccga agacagggaa 3060

aattgtgtca atgcccttcc acctaaaacc aaaactctac ccagatttca tggggaaaga 3120

agactaccaa acttacaagt cgaacaaaat cttgggtcgt ctttacagaa gggtaaaaca 3180

agtgtatgat gaagatgcag aagcttcttc agaagaaatc ccagacccta gtgacatccc 3240

ttatgacact gatcttgaaa tacctggatt cgcagagttg atccccgagg catggggtca 3300

caaatgttct tacgacgggc agctcattgg tcttcttggg caatataagg tccagaaaga 3360

ggaagagatt gtgacgggcc atatctggtc gatgccgaaa tacgctagta agaaacaagg 3420

ggaattgaaa gaaagattga agcattctta taactccctc aaaaaggagt tcagaaaagt 3480

attcgaggaa acaatcccgg accaggaaaa tctcagcgat gaggaaaaaa acatcttgta 3540

tgagaagaag gcttcagctt ggtatcatgt cacttaccat cccgagtggg tgaagaagtc 3600

tttagagctg caagatccag atgagtcgtc tagtcatgcg gtgatgctga gttttgcttg 3660

gattgcagct gattaccttg caagaatcaa agtccggtca cgggaaatgg aaaatatcga 3720

ctcagccaag cctgttgatt ctttggccaa gtttcttgct caacgtctct aaaaggtaat 3780

gttcgaagcc gtaaggccac tctgccatgt atctgtggaa attcacagat actccaatat 3840

ttgtaagcta gttttgaatg tgctattctg gaaactttgt gtgtgatgaa ttcaggattt 3900

aaacagctca actgtttgcc gttgtttgtg gttatgtatt ttactaaact ttcgtttcaa 3960

ttttattaca tgggttgata a 3981

<210> 8
<211> 3988
<212> DNA
<213> Camelina sativa

<400> 8
tgggagagag agagtgggag aagtcaagaa taatcatgac gcatggtttc actttcagct 60

atggcgattt ctccttccta atttagcgcc tactcactta ctaatcttct ctccggtctc 120

cgcgttgctt ctgtttcgtc ttcagtatcg attccgttca ctgttggatt agggctttac 180

ggagaatggg gtcagaggga aacatgaaga actcggttgt gacacaagta agcattggtg 240

gatttggtga gtccactact gcaaaacaac ttacagatta cctcgaagat gaagtaggac 300

ttgtttggcg atgcagatta aaaaactctt ggactccccc tgggtcttat cctaatttcg 360

aaatcgcaga cacttcaaaa atccccaaaa tcaatgatta caagaaagtg gagcctcatg 420

cgtttgtcca tttcgcgctt cctgaatcaa cagcacgtgc catggatgct gcagggcaat 480

gcaagctcat ccttgatggc cagcctttaa aggtcagctt ggggcctaag aacccatata 540

cacttaacca gagaagaaga acgactactc catataagtt gactggtgtt tcggtcgaga 600

ttgggacttt ggtttcccgg gatgagtttc ttgtttcttg gagagctgaa ggggttgatt 660

tccttgtaga tccttttgac aacacttgca agttttgttt caggaagagc actgcgttct 720

ctttcaagga tgcagtgatg catgctgtga taaattgtga ttataagttg gagttgttgg 780

tgagagacat aagaacagtt aggcagtata agaatttgca tggctttgtg cttcttttgc 840

agctggcttc ttcaccccgt gtctggtaca gaactgctga tgatgatatt tacgagactg 900

ttcctttcga tctcttggat gatgatgacc cttggatccg taccactgat tttacacaag 960

ttggggcgat cggtcgatgt ctttcttatc gagtgctcat atctcctcgg tatgagaaga 1020

agttgacaac ggcattagat tatctaagga tgcggagggt gcaagaggaa cgtgtgaggt 1080

ggcctcctcg gatccgtgat gagccttgtt ttggggagcc tgtgtcagat catttcttct 1140

gcattcatca caaggaagga atctcctttg agatcatgtt cctagtaaac tcggtattgc 1200

acagaggggt ttttaaccag tttcagttga ctgagcgttt ctttgatctt ctcagaaacc 1260

aatccagaga tgtcaatata gcctctctca agcatctctg tacctataaa agaccagttt 1320

ttgatgcgta caagaggctg aagcttgttc aggaatggat tcagaaaaat cccaagcttt 1380

tagggagtca tgaacaatct gaggatatct ctgagatcag aaggttagtg attaccccaa 1440

ccagagccca ttgcctaccc ccagaagttg agctctccaa cagggtactc aggaaataca 1500

aagctgttgc ggaaagattt ttgcgcgtaa cattcatgga tgaaagtatg cagaccataa 1560

attcgaatgt tctctcttac tttgtcgctc ctattgtgaa ggatctgaca tcaagctctt 1620

tctctcagaa gacttacgtt ttcaaaagag tgaagagcat attaaccgat gggtttaaac 1680

tatgtggtag aaaatatagt tttctagcat tctcagccaa tcaactgaga gaccgctctg 1740

cttggttctt tgctgaagac gggaaaacaa gagtgtcaga tataaaaaca tggatgggga 1800

agttcaaaga caagaatgtg gcaaaatgtg ctgctaggat gggactgtgc ttctcctcca 1860

catatgccac tgtagatgtc atgcttcacg aggttgacac cgagcttcca gacattgaga 1920

gaaatgggta tgttttctct gatggaattg gtacaatcac acctgacctc gctgacgaag 1980

taatggagaa acttaggttg gatgtgcact acaccccttg tgcttatcag atacgttacg 2040

caggtttcaa aggagttgtt gctcgttggc catcaaaagg tgatggaatc cggctagccc 2100

ttcgagacag tatgaagaag ttcaattcca aacatattat cttagagatc tgttcctgga 2160

cgaggtttca acctggattc ttgaaccggc agattattac tctcctttct gtactaggtg 2220

ttccggatga aatattctgg gatatgcagg aatccatgct ctgtaaactg aaccgcatcc 2280

ttgatgatac cgatgttgca tttgaagttc taacagcatc atgtgctgaa caggggaaca 2340

ctgcagctat catgctaagt gcaggtttca aaccaaaaac tgagccacat ctacgtggaa 2400

tgctgtcttc agtcagaatt gcacaactct ggggtctcag agaaaaatct cgtatttttg 2460

ttacttcagg aaggtggctg atgggttgcc ttgacgaagc agggatactt gaacagggac 2520

aatgctttat ccaagtctca aaaccgtctt tagaaaattg tttctccaaa catgggtctc 2580

gttttaagga gacaaagaca gatctgcaag tagttaaagg ctatgtagcc attgccaaga 2640

atccttgtct tcacccaggg gatgtaagga ttttagaagc tgttgatgta ccccagctgc 2700

atcacatgta tgactgcctt attttccctc agaaaggtga taggccccat acaaacgaag 2760

cttctggcag tgaccttgac ggggacctgt actttgtggc ttgggatcag aaactcatcc 2820

ctcccagcaa aaaaagcttt ccggccatgc attatgatgc agctgaagag aagagtttag 2880

gccgtgctgt caaccaccag gatataatag atttctttgc aagaaacatg gcaaatgagc 2940

atttgggtac aatttgcaat gcacacgtcg ttcatgctga tagaagtgag tatggagcca 3000

tggacgaaga atgtatgcta ctggcagaat tagctgccac tgcagttgat ttcccgaaga 3060

cagggaaaat tgtgtcaatg cccttccacc taaaaccaaa actctaccca gatttcatgg 3120

ggaaagaaga ctaccaaact tacaagtcga acaaaatctt gggtcggctt tacagaaggg 3180

taaaggaagt gtatgatgaa gatgcagaag cttcctcaga agaaatccca gacccaagtg 3240

acatccctta tgacactgat cttgaaatac ctggattcgc agatctgatc cccgaggcat 3300

ggggtcacaa atgttcttac gacgggcagc tcattggtct tcttgggcaa tataaggtcc 3360

agaaagagga agagattgtg acgggccata tctggtcgat gcctagatac gctagtaaga 3420

aacaagggga attgaaagaa agattgaagc attcttataa ctccctcaaa aaggagttca 3480

gaaaagtatt cgagggaaca atcccggacc aggaaaatct cagcgatgag gaaaaaaaca 3540

tcttgtatga gaaaaaggct tcagcttggt atcatgtcac ttaccatccc gagtgggtga 3600

agaagtcttt agagctgcaa gatccagatg agtcgtctag tcatgcggtg atgctgagtt 3660

ttgcttggat tgcagctgat tatcttgcaa gaatcaaaat ccggtcacgg gaaatgggaa 3720

atatcgactc agccaagcct gttgattctt tggccaagtt tcttgctcaa cgtctctaaa 3780

aggtaatgtt cgaagccgta aggccactct gccatgtatc tgtggaaatt cacagatact 3840

ccaatatatg taagctagtt ttgaatgtgc tattctggaa actttgtgtg tgatgaattc 3900

aggatttaaa cagctcaact gtttgccgtt gtttgtcgtt atgtatttta ctaaactttc 3960

gtttcaattt tattacatgg gttgataa 3988

<210> 9
<211> 960
<212> DNA
<213> Cauliflower mosaic virus

<400> 9
ccagtacgca cgattcaagg cttgcttcac aaaccaaggc aagtaataga gattggagtc 60

tctaaaaagg tagttcccac tgaatcaaag gccatggagt caaagattca aatagaggac 120

ctaacagaac tcgccgtaaa gactggcgaa cagttcatac agagtctctt acgactcaat 180

gacaagaaga aaatcttcgt caacatggtg gagcacgaca cacttgtcta ctccaaaaat 240

atcaaagata cagtctcaga agaccaaagg gcaattgaga cttttcaaca aagggtaata 300

tccggaaacc tcctcggatt ccattgccca gctatctgtc actttattgt gaagatagtg 360

gaaaaggaag gtggctccta caaatgccat cattgcgata aaggaaaggc catcgttgaa 420

gatgcctctg ccgacagtgg tcccaaagat ggacccccac ccacgaggag catcgtggaa 480

aaagaagacg ttccaaccac gtcttcaaag caagtggatt gatgtgataa catggtggag 540

cacgacacac ttgtctactc caaaaatatc aaagatacag tctcagaaga ccaaagggca 600

attgagactt ttcaacaaag ggtaatatcc ggaaacctcc tcggattcca ttgcccagct 660

atctgtcact ttattgtgaa gatagtggaa aaggaaggtg gctcctacaa atgccatcat 720

tgcgataaag gaaaggccat cgttgaagat cctctgccga cagtggtccc aaagatggac 780

ccccacccac gaggagcatc gtggaaaaag aagacgttcc aaccacgtct tcaaagcaag 840

tggattgatg tgatatctcc actgacgtaa gggatgacgc acaatcccac tatccttcgc 900

aagacccttc ctctatataa ggaagttcat ttcatttgga gaggacacgc tgaaatcacc 960

<210> 10
<211> 509
<212> DNA
<213> Cassava vein mosaic virus

<400> 10
agaaggtaat tatccaagat gtagcatcaa gaatccaatg tttacgggaa aaactatgga 60

agtattatgt gagctcagca agaagcagat caatatgcgg cacatatgca acctatgttc 120

aaaaatgaag aatgtacaga tacaagatcc tatactgcca gaatacgaag aagaatacgt 180

agaaattgaa aaagaagaac caggcgaaga aaagaatctt gaagacgtaa gcactgacga 240

caacaatgaa aagaagaaga taaggtcggt gattgtgaaa gagacataga ggacacatgt 300

aaggtggaaa atgtaagggc ggaaagtaac cttatcacaa aggaatctta tcccccacta 360

cttatccttt tatatttttc cgtgtcattt ttgcccttga gttttcctat ataaggaacc 420

aagttcggca tttgtgaaaa caagaaaaaa tttggtgtaa gctattttct ttgaagtact 480

gaggatacaa cttcagagaa atttgtaag 509

<210> 11
<211> 1161
<212> DNA
<213> Custom

<400> 11
atggcggata cagctagagg aacccatcac gatatcatcg gcagagacca gtacccgatg 60

atgggccgag accgagacca gtaccagatg tccggacgag gatctgacta ctccaagtct 120

aggcagattg ctaaagctgc aactgctgtc acagctggtg gttccctcct tgttctctcc 180

agccttaccc ttgttggaac tgtcatagct ttgactgttg caacacctct gctcgttatc 240

ttcagcccaa tccttgtccc ggctctcatc acagttgcac tcctcatcac cggttttctt 300

tcctctggag ggtttggcat tgccgctata accgttttct cttggattta caagtacgca 360

acgggagagc acccacaggg atcagacaag ttggacagtg caaggatgaa gttgggaagc 420

aaagctcagg atctgaaaga cagagctcag tactacggac agcaacatac tggtggggaa 480

catgaccgtg accgtactcg tggtggccag cacactactg aagatttttt gcaaaagcaa 540

caatatggaa tttcttctaa gttttggcct tttggtgaag tttttaacgc caccagattt 600

gcatctgttt atgcttggaa caggaagaga atcagcaact gtgttgctga ttattctgtc 660

ctatataatt ccgcatcatt ttccactttt aagtgttatg gagtgtctcc tactaaatta 720

aatgatctct gctttactaa tgtctatgca gattcatttg taattagagg tgatgaagtc 780

agacaaatcg ctccagggca aactggaaag attgctgatt ataattataa attaccagat 840

gattttacag gctgcgttat agcttggaat tctaacaatc ttgattctaa ggttggtggt 900

aattataatt acctgtatag attgtttagg aagtctaatc tcaaaccttt tgagagagat 960

atttcaactg aaatctatca ggccggtagc acaccttgta atggtgttga aggttttaat 1020

tgttactttc ctttacaatc atatggtttc caacccacta atggtgttgg ttaccaacca 1080

tacagagtag tagtactttc ttttgaactt ctacatgcac cagcaactgt ttgtggacct 1140

aaaaagtcta ctaatttgtg a 1161